# EUROPEAN PATENT SPECIFICATION

(11) **EP 3 440 219 B1**
(45) Date of publication and mention of the grant of the patent: **01.09.2021**
(21) Application number: 17717362.2
(22) Date of filing: 07.04.2017
(51) Int. Cl.: C12Q 1/68

(54) **METHODS AND KITS FOR PREDICTING THE SENSITIVITY OF A SUBJECT SUFFERING OF RENAL CANCER TO CANCER TREATMENT**
VERFAHREN UND KITS ZUR VORHERSAGE DER EMPFINDLICHKEIT EINES UNTER NIERENKREBS LEIDENDEN SUBJEKTS AUF EINEN INHIBITOR VON TYROSINKINASEREZEPTOREN
PROCÉDÉS ET KITS POUR PRÉDIRE LA SENSIBILITÉ D'UN SUJET ATTEINT DE CANCER RÉNAL À UN INHIBITEUR DES RÉCEPTEURS DE LA TYROSINE KINASE

(30) Priority: 08.04.2016 EP 16305411
(43) Date of publication of application: 13.02.2019
(73) Proprietor: Centre National de la Recherche Scientifique, 75016 Paris (FR); INSERM (Institut National de la Santé et de la Recherche Médicale), 75013 Paris (FR); Université Côte d'Azur, 06100 Nice (FR); Centre Antoine Lacassagne, 06189 Nice Cedex 2 (FR)
(72) Inventor: PAGES, Gilles, 98000 Monaco (MC); ETTAICHE, Marc, 06790 Aspremont (FR); CHAMOREY, Emmanuel, 06000 Nice (FR); DUFIES, Maeva, 06200 Nice (FR); GIULIANO, Sandy, 06100 Nice (FR)
(74) Representative: Regimbeau
(86) International application number: PCT/EP2017/058317
(87) International publication number: WO 2017/174759

(56) References cited:
- WO-A1-2015/050500
- HAI T TRAN ET AL: "Prognostic or predictive plasma cytokines and angiogenic factors for patients treated with pazopanib for metastatic renal-cell cancer: a retrospective analysis of phase 2 and phase 3 trials", THE LANCET ONCOLOGY, vol. 13, no. 8, 1 August 2012 (2012-08-01) , pages 827-837, XP055371640, AMSTERDAM, NL ISSN: 1470-2045, DOI: 10.1016/S1470-2045(12)70241-3
- R. GREPIN ET AL: "The CXCL7/CXCR1/2 Axis Is a Key Driver in the Growth of Clear Cell Renal Cell Carcinoma", CANCER RESEARCH, vol. 74, no. 3, 12 December 2013 (2013-12-12), pages 873-883, XP055371270, US ISSN: 0008-5472, DOI: 10.1158/0008-5472.CAN-13-1267

## Description

### FIELD OF THE INVENTION

The present invention relates to the subject-matters of claims 1 to 14.

The present disclosure relates to a method of predicting or monitoring the sensitivity of a subject having a tumor to cancer treatment (also herein identified as "chemotherapy"), in particular a method of predicting or monitoring the sensitivity of a subject having renal cell carcinoma (RCC) to an inhibitor of a tyrosine kinase receptors or an antibody selected from nivolumab, atezolizumab and avelumab, to a method of selecting an appropriate treatment of cancer, to a method of screening or identifying a compound suitable for improving the treatment of a cancer, and to corresponding kits. The method of predicting or monitoring the sensitivity of a subject having renal cell carcinoma (RCC) to an inhibitor of a tyrosine kinase receptor or to an antibody selected from nivolumab, atezolizumab and avelumab typically comprises a step a) of determining, in a biological sample from said subject, the expression level of at least CXCL5 or CXCL7, preferably of CXCL5 and CXCL7, and when the expression level(s) is(are) determined, a step b) of comparing said expression level(s) to a reference expression level, thereby assessing or monitoring whether the subject having ccRCC is responsive or resistant to the inhibitor of a tyrosine kinase receptor or antibody.

### BACKGROUND OF THE INVENTION

Renal cell carcinoma (RCC, also known as hypernephroma) is a renal cancer that originates in the lining of the proximal convoluted tubule, the very small tubes in the kidney that filter the blood and remove waste products. Clear cell metastatic renal cell carcinoma (ccRCC) is the most common type of kidney cancer in adults, responsible for approximately 80% of cases (Mulders PF et al., 2008, Ned Tijdschr Geneeskd, 152: 376). It is also known to be the most lethal of all the genitourinary tumors. Initial treatment is most commonly a radical or partial nephrectomy and remains the mainstay of curative treatment (Rini BI et al., 2008). Where the tumor is confined to the renal parenchyma, the 5-year survival rate is 60-70%, but this is lowered considerably where metastases have spread. It is resistant to radiation therapy and chemotherapy, but some cases respond to immunotherapy. Targeted cancer therapies such as sunitinib, temsirolimus, bevacizumab, interferon-alpha, and sorafenib have improved the outlook for RCC (progression-free survival), although they have not yet demonstrated improved survival.

Anti-angiogenic therapies have been used in clear cell renal cancers (ccRCC, the most common renal cancer) with mitigated results (Escudier *et al.,* 2010). Although these treatments increase progression-free survival of patients, they do not impact the overall survival except in some extremely rare cases. One hypothesis that could explain this semi failure is the production by the tumor cells or cells of the tumor microenvironment of angiogenesis factors redundant to VEGF or its receptors (main therapeutic targets of current treatments). The inventors have shown that the ELR + CXCL cytokines are more relevant than VEGF as prognostic markers of survival in clear cell metastatic renal cell carcinoma (ccRCC) (Grépin *et al.,* 2014; Grépin *et al.,* 2012). The importance of these cytokines has led them to develop monoclonal antibodies targeting concomitantly the three most relevant "prognostic" cytokines in the context of RCC: CXCL1, CXCL5, CXCL7 and CXCL8 (WO2014/184384). Contrary to anti-VEGF antibodies, these antibodies significantly reduce tumor growth in experimental animal models. These antibodies are as effective as sunitinib to block experimental tumor growth in mice. The heterogeneity of response to sunitinib, the current therapeutic standard (Motzer et al., N Engl J Med, 2007) is a real problem. Identifying patients likely to respond to treatment represents a therapeutic challenge to limit the administration of ineffective toxic products. Thus, the discovery of predictive markers of response to treatment would avoid a waste of time at diagnostic and would anticipate gains treatment. The clinician would therefore choose an alternative treatment among those available (usually administered in the second or third line treatment) (Motzer et al., Lancet Oncol 2013; Motzer et al., N Engl J Med, 2013; Motzer et al., Lancet 2008).

The accurate selection of the patients capable of responding to a particular chemotherapy is a solution for them to receive the most appropriate therapy as soon as possible and typically as soon as they are diagnosed.

WO2015/050500 discloses a 10-gene prognostic therapy assay to predict benefit from TKI therapy of ccRcc renal cancer. Hai tran et al. (The Lancet Oncology, vol.13, n°8, 1 August 2012, pages 827-837) discloses that cytokine profiles provide markers predictive of the response to TKI pazopanib in patients with real cell carcinoma. But the teaching of these documents, on intra-tumoral samples would not suggest to use blood fluid samples, in particular a blood, a serum, a plasma or derivative thereof and follow the variation of said markers CxCL5 and/or CxCL7 to assess sensitivity or resistance to TKI treatment.

### SUMMARY OF THE DISCLOSURE AND THE INVENTION

The present invention concerns the subject matters as claimed in claims 1-14.

The present invention is based on the discovery by inventors that CXCL5 is a predicting marker of response to a chemotherapeutic treatment of cancer, in particular renal cancer, more specifically renal cell carcinoma (RCC), in particular clear cell renal cell carcinoma (ccRCC) or metastatic ccRCC. Inventors further discover the value of assessing CXCL7 instead of CXCL5 or in addition to CXCL5. The present disclosure includes methods and kits for predicting or assessing the response of a subject having a tumor or cancer, in particular a renal cancer as herein identified, to a particular chemotherapeutic treatment ("chemotherapy"), in particular to an inhibitor of a tyrosine kinase receptor (also herein identified as a "tyrosine kinase inhibitor"), typically to an inhibitor of tyrosine kinase receptors, but also to an antibody selected from nivolumab, atezolizumab and avelumab, using anyone of these biomarkers or a combination thereof.

In the context of the present disclosure, the expressions "chemotherapeutic treatment of cancer" and "chemotherapy" generally designate a "cancer treatment" and for example cover any targeted cancer therapy such as those involving an inhibitor of a tyrosine kinase receptor or a (monoclonal) antibody.

A first method herein described in the disclosure is an *in vitro* or *ex vivo* method of assessing (or predicting) or monitoring the sensitivity of a subject having a cancer to a chemotherapy, typically the sensitivity of a subject having a renal cancer, more specifically a renal cell carcinoma (RCC), in particular a clear cell renal cell carcinoma (ccRCC) or metastatic ccRCC, to a chemotherapy, which method comprises a step a) of determining, in a biological sample from said subject, the expression level of CXCL5 or CXCL7, and, when the CXCL5 or CXCL7 expression level is determined, a step b) of comparing said CXCL5 or CXCL7 expression level to a CXCL5 or CXCL7 reference expression level, thereby assessing or monitoring whether the subject having a tumor, typically a renal cancer, is responsive or resistant to the chemotherapy.

A particular method herein described in the disclosure is a method, typically an *in vitro* or *ex vivo* method, of selecting an appropriate chemotherapeutic treatment of cancer for a subject having a cancer, typically a renal cancer, more specifically a renal cell carcinoma (RCC), in particular a clear cell renal cell carcinoma (ccRCC) or metastatic ccRCC, which method comprises a step a) of determining, in a biological sample of said subject, the expression level(s) of CXCL5 and/or CXCL7, and a step b) of comparing said level to corresponding (CXCL5 and/or CXCL7) reference expression level(s), CXCL5 and/or CXCL7 expression level(s) identical to or below the (CXCL5 and/or CXCL7) reference expression level(s) being the indication that an inhibitor of a tyrosine kinase receptor, or an antibody selected from nivolumab, atezolizumab and avelumab, will not be efficient alone against cancer in the subject, and a step c) of selecting an appropriate chemotherapeutic treatment of cancer in the subject; and, on the contrary, CXCL5 and/or CXCL7 expression level(s) above the (CXCL5 and/or CXCL7) reference expression level(s) being the indication that an inhibitor of a tyrosine kinase receptor or an antibody selected from nivolumab, atezolizumab and avelumab will be efficient alone against cancer in the subject (said inhibitor of a tyrosine kinase receptor or antibody being an appropriate chemotherapeutic treatment of the cancer in the subject).

Also herein described in the disclosure is a method for screening or identifying a compound suitable for improving the treatment of a cancer as herein identified in a subject having such a cancer, said method comprising determining the ability of a test compound to modify the expression of CXCL5 and/or of CXCL7, or compensate an abnormal expression thereof.

A further aspect of the disclosure relates to the use of a kit i) for assessing or monitoring the sensitivity or resistance of a subject having a cancer, in particular a renal cancer, to a chemotherapy, and/or ii) for determining the potential toxicity of said chemotherapy in a subject having a cancer, in particular a renal cancer, wherein the kit comprises detection means selected from the group consisting of at least one antibody specific to CXCL5 or CXCL7, preferably an antibody specific to CXCL5 and an antibody specific to CXCL7 or an antibody recognizing specifically both CXCL5 and CXCL7; a molecule allowing the antibody(ies) detection; and, optionally, a leaflet providing the CXCL5 and/or CXCL7 respective reference expression level(s), corresponding typically to the reference expression level(s) in a control population.

### DETAILED DESCRIPTION OF THE DISCLOSURE AND THE INVENTION

CXCL5 is a small cytokine belonging to the CXC chemokine family that is also known as epithelial-derived neutrophil-activating peptide 78 (ENA-78). A typical CXCL5 amino acid sequence has the sequence of SEQ ID NO: 1. It is produced following stimulation of cells with the inflammatory cytokines interleukin-1 or tumor necrosis factor-alpha. Expression of CXCL5 has also been observed in eosinophils, and can be inhibited with the type II interferon IFN-y. This chemokine stimulates the chemotaxis of neutrophils possessing angiogenic properties. It elicits these effects by interacting with the cell surface chemokine receptor CXCR2. The gene for CXCL5 is encoded on four exons and is located on human chromosome 4 among several other CXC chemokine genes. Antibodies directed against CXCL5 have been described and are available in the art. The skilled person can for example use the CXCL5 specific antibody generated by R&D Systems (R&D Systems' reference: DY254).

CXCL7 is a small cytokine belonging to the CXC chemokine family. It is an isoform of Beta-Thromboglobulin or Pro-Platelet basic protein (PPBP). A typical nucleic acid sequence encoding CXCL7 is SEQ ID NO: 2. It is a protein that is released in large amounts from platelets following their activation. It stimulates various processes including mitogenesis, synthesis of extracellular matrix, glucose metabolism and synthesis of plasminogen activator. Antibodies directed against CXCL7 have been described and are available in the art. The skilled person can for example use one of the CXCL7 specific antibodies generated by Peprotech (cf. for example Peprotech' reference 900-K40).

Preferred CXCL5 and CXCL7 antibodies targeting concomitantly CXCL1, CXCL5, CXCL7 and CXCL8 are antibodies 12A10-13 and 35B11-8 described in WO2014/184384 (produced respectively by hybridoma corresponding to CNCM deposit No.: 1-4617 and 1-4618). Any antibodies comprising CDR sequences of antibodies 12A10-13 and/or 35B11-8 (selected from SEQ ID NO: 3-14 and any combination thereof) as well as humanized versions thereof can advantageously be used in the context of the invention.

In the present disclosure, the cancer is a cancer that is conventionally treated with one of the following cancer therapy: immunotherapy, specific kinase inhibitor-based therapy, antiangiogenic agent based-therapy, antibody-based therapy and surgery. In the context of the present disclosure, the anti-cancer drug or agent (also herein generally identified as "chemotherapeutic drug or agent" or "chemotherapy") encompasses among others kinase inhibitors, antiangiogenic agents (such as sorafenib), cytokine, as well as monoclonal antibodies (typically an antibody selected from nivolumab, atezolizumab and avelumab), and any combination thereof used in the treatment of cancer.

Cancers sensitive to a specific kinase inhibitor-based therapy are conventionally treated using a compound selected for example from a tyrosine kinase inhibitor, a serine kinase inhibitor and a threonine kinase inhibitor.

The cancer or tumor may be any kind of cancer or neoplasia. The cancer is typically selected from a carcinoma, a sarcoma, a lymphoma, a melanoma, a paediatric tumour and a leukaemia tumour.

The cancer is preferably selected from a carcinoma, a melanoma and a paediatric tumour.

The cancer is even more preferably a carcinoma. A particular carcinoma is a renal cancer, for example a clear cell renal cancer (ccRCC), typically a renal cancer, such as the metastatic renal cancer, conventionally treated with a cytokine [typically interferon alpha or interleukin 2]; an anti-angiogenic drug for example selected from a tyrosine kinase inhibitor, a serine kinase inhibitor [in particular a mTOR inhibitor, typically everolimus or temsirolimus] and a threonine kinase inhibitor [typically a tyrosine kinase inhibitor such as sunitinib, axitinib, pazopanib, sorafenib; or the bevacizumab (VEGF trap also identified as aflibercept)]; or a combination thereof, typically the combination of a cytokine and of an anti-angiogenic drug, preferably the combination of interferon alpha and bevacizumab.

In the context of the present disclosure, the patient or subject is a mammal. In a particular embodiment, the mammal is a human being, whatever its age or sex. The patient typically has a tumor. Unless otherwise specified in the present disclosure, the tumor is a cancerous or malignant tumor. In a particular aspect, the subject is a subject who has not been previously exposed to a treatment of cancer or a subject who has received the first administration of a chemotherapeutic drug. In another particular aspect, the subject is a subject who has undergone at least partial resection of the cancerous tumor. Another particular subpopulation of subjects is composed of subjects having metastases.

In the context of renal cancer, a particular subpopulation of subjects is composed of high or intermediate risk of relapse subjects according to the Motzer classification (Motzer RJ et, al., 1999, J Clin Oncol, 17:2530). These subjects are distinct from the subjects classified as low risk or relapse subjects according to said classification.

Implementations of the methods of the disclosure involve obtaining a (biological) sample from a subject. The sample is preferably a fluid sample and may include serum, blood, plasma, lymphatic fluid, spinal fluid, pleural effusion, ascites, or a combination thereof. The sample of the invention is typically selected from a blood sample, a serum sample, or a plasma sample. A preferred sample of the invention is a plasma sample such as a plasma sample free of (or devoid of) blood platelets. Another preferred sample is a fluid sample without cells.

A method according to the present disclosure is an *in vitro* or *ex vivo* method of assessing (or predicting) or monitoring the sensitivity of a subject having a cancer such as herein described, in particular a renal cancer, to a chemotherapy, in particular to an inhibitor of a tyrosine kinase receptor (also herein identified as a "tyrosine kinase inhibitor"), or to an antibody selected from nivolumab, atezolizumab and avelumab, which method comprises a step a) of determining, in a biological sample from said subject, the presence, absence or expression level of CXCL5 or CXCL7, and, when the CXCL5 or CXCL7 expression level is determined, a step b) of comparing said CXCL5 or CXCL7 expression level to a (CXCL5 or CXCL7) reference expression level, thereby assessing or monitoring whether the subject having a cancer is responsive or resistant to the chemotherapy.

In a preferred embodiment of the invention, when the method comprises a step a) of determining, in a biological sample from said subject, the presence, absence or protein expression level of CXCL5, said method further comprises a step a') of determining, in a biological sample from said subject, the protein expression level of CXCL7 and, when the CXCL7 protein expression level is determined, a step b') of comparing said CXCL7 protein expression level to a CXCL7 reference protein expression level.

In another aspect of the disclosure, when the method comprises a step a) of determining, in a biological sample from said subject, the presence, absence or expression level of CXCL7 said method further comprises a step a') of determining, in a biological sample from said subject, the expression level of CXCL5 and, when the CXCL5 expression level is determined, a step b') of comparing said CXCL5 expression level to a CXCL5 reference expression level.

By "sensitivity" or "responsiveness" is intended herein the likelihood that a patient will respond to a chemotherapeutic treatment.

By "resistant" is intended herein the likelihood that a patient will not respond to a chemotherapeutic treatment.

Predictive methods of the invention can be used clinically to make treatment decisions by choosing as soon as possible the most appropriate treatment modalities for a particular patient.

If the subject is identified, using a method according to the present invention, as resistant to a particular treatment of cancer, the method advantageously further comprises a step of selecting a distinct chemotherapeutic treatment, typically involving a "compensatory molecule", to be used instead of or in combination with the originally preselected chemotherapeutic drug or with a distinct chemotherapeutic drug, as the appropriate therapeutic treatment of cancer for the subject.

Preferably, the step of determining the presence, absence or expression level of at least CXCL5 and/or CXCL7 in a biological sample of the subject is performed before any chemotherapeutic treatment step, i.e. before any administration to the subject of a chemotherapy for treating the subject's cancer. Less preferably but also possible, this step can be performed after the first administration of a chemotherapeutic drug to the subject. This step can be performed before any tumor surgical resection. It can also be performed on a subject who has undergone at least partial resection of the cancerous tumor.

In a particular embodiment, the method according to the present invention is an *in vitro* or *ex vivo* method of assessing, predicting or monitoring the sensitivity of a subject having a renal cancer, preferably a clear cell renal cell carcinoma (ccRCC), preferably a metastatic ccRCC, and the chemotherapy is a kinase inhibitor-based therapy selected for example from a tyrosine kinase inhibitor, a serine kinase inhibitor (such as a mTOR inhibitor selected from everolimus and temsirolimus) and a threonine kinase inhibitor, preferably a tyrosine kinase inhibitor, more specifically an inhibitor of a tyrosine kinase receptor, preferably selected from sunitinib, axitinib, pazopanib, and sorafenib. In a preferred embodiment, the inhibitor of a tyrosine kinase receptor is sunitinib.

In another aspect, the method according to the disclosure is an *in vitro* or *ex vivo* method of assessing, predicting or monitoring the sensitivity of a subject having a renal cancer, preferably a clear cell renal cell carcinoma (ccRCC), preferably a metastatic ccRCC, and the chemotherapy is an antibody selected from nivolumab, atezolizumab and avelumab.

Herein described in the disclosure is typically an *in vitro* or *ex vivo* method wherein the cancer is a renal cancer as herein described, in particular a metastatic renal cancer, and wherein the chemotherapy is a kinase inhibitor-based therapy, in particular an inhibitor of a tyrosine kinase receptor as herein identified, or a (monoclonal) antibody-based therapy, and the method comprises a step a) of determining, in a biological sample of the subject, the expression level of CXCL5, and optionally the expression level of CXCL7, and a step b) of comparing said CXCL5 and optionally CXCL7 expression level(s) to a CXCL5 and optionally CXCL7 reference expression level(s) or reference value(s), a CXCL5 and optionally CXCL7 expression level(s) (as measured in the biological sample) identical to or below their respective reference expression level(s) being indicative of resistance of the subject to the chemotherapy whereas a CXCL5 and optionally CXCL7 expression level(s) (as measured in the biological sample) above their respective reference expression level(s) being indicative of sensitivity of the subject to the chemotherapy, or in other words being indicative that the chemotherapy will be efficient in said subject.

A particular method of the disclosure comprises a step of determining, in a biological sample from the subject, the respective expression levels of CXCL5 and CXCL7, and, when the CXCL5 and CXCL7 expression levels are determined, a step of comparing said CXCL5 and CXCL7 expression levels respectively to CXCL5 and CXCL7 reference expression levels, thereby assessing whether the subject is sensitive or resistant to the chemotherapy, in particular to the tyrosine kinase inhibitor or to the antibody (typically selected from nivolumab, atezolizumab and avelumab).

Typically, the "reference value" or "reference expression level" is the level of CXCL5 or CXCL7 in a control sample derived from one or more subjects (reference population) having a cancer, in particular a renal cancer, and is typically the median value when obtained from the reference population.

As an example of the invention, when the patient is bearing a metastatic ccRCC cancer, the candidate chemotherapeutic drug (herein identified as chemotherapy) being an inhibitor of a tyrosine kinase receptor such as herein described, in particular sunitinib, the CXCR5 reference protein expression level is of about 100 pg/ml and the CXCR7 reference protein expression level is of about 250 ng/ml.

In a particular embodiment of the invention, when the inhibitor of a tyrosine kinase receptor is sunitinib, a CXCL5 protein expression level identical to or below 100 pg/ml together with a CXCL7 protein expression level identical to or below 250 ng/ml is indicative of a resistance of the subject to sunitinib, and a CXCL5 protein expression level above 100 pg/ml together with a CXCL7 protein level above 250 ng/ml is indicative of a sensitivity of the subject to sunitinib.

In some examples of the disclosure, identification of CXCL5 and/or CXCL7 involves use of at least one CXCL5 and/or CXCL7 polypeptide binding agent.

The polypeptide is, in particular examples, an antibody. In further examples in relation with CXCL5 or CXCL7, the antibody is a monoclonal antibody. Preferably, the CXCL5 or CXCL7 antibody is selected from a specific CXCL5 or CXCL7 antibody of the art. The antibody can be bi-specific, recognizing two different epitopes. The antibody, in some examples, immunologically binds to more than one epitope from the same CXCL5 or CXCL7 polypeptide.

A CXCL5 polypeptide binding agent that is a polypeptide may also include all or part of CXCR2, which is a receptor for CXCL5 polypeptides.

In some examples of the disclosure, the soluble CXCL5 or CXCL7 polypeptide binding agent is an aptamer.

In some examples of the disclosure, the CXCL5 or CXCL7 binding agent is labeled. In further examples, the label is radioactive, fluorescent, chemiluminescent, an enzyme, or a ligand. It is also specifically contemplated that a binding agent is unlabeled, but may be used in conjunction with a detection agent that is labeled. A detection agent is a compound that allows for the detection or isolation of itself so as to allow detection of another compound that binds, directly or indirectly. An indirect binding refers to binding among compounds that do not bind each other directly but associate or are in a complex with each other because they bind the same compounds or compounds that bind each other.

Other examples of the disclosure involve a second CXCL5 or CXCL7 polypeptide binding agent in addition to a first CXCL5 or CXCL7 polypeptide binding agent. The second binding agent may be any of the entities discussed above with respect to the first binding agent, such as an antibody. It is contemplated that a second antibody may bind to the same of different epitopes as the first antibody. It is also contemplated that the second antibody may bind the first antibody or another epitope than the one recognized by the first antibody.

As discussed earlier, binding agents may be labeled or unlabeled. Any CXCL5 or CXCL7 polypeptide binding agent used in methods of the disclosure may be recognized using at least one detection agent. A detection agent may be an antibody that binds to a CXCL5 or CXCL7 polypeptide binding agent, such as an antibody. The detection agent antibody, in some examples, binds to the Fc-region of a binding agent antibody. In further examples, the detection agent is biotinylated, which is incubated, in additional embodiments, with a second detection agent comprising streptavidin and a label. It is contemplated that the label may be radioactive, fluorescent, chemiluminescent, an enzyme, or a ligand. In some cases, the label is an enzyme, such as horseradish peroxidase.

The present disclosure also covers methods involving using an ELISA assay to identify a CXCL5 or CXCL7 polypeptide. In some examples, the ELISA assay is a sandwich assay. In a sandwich assay, more than one antibody will be employed. Typically ELISA method can be used, wherein the wells of a microtiter plate are coated with a set of antibodies which recognize the protein of interest. A sample containing or suspected of containing the protein of interest is then added to the coated wells. After a period of incubation sufficient to allow the formation of antibody-antigen complexes, the plate(s) can be washed to remove unbound moieties and a detectably labelled secondary binding molecule added. The secondary binding molecule is allowed to react with any captured sample marker protein, the plate washed and the presence of the secondary binding molecule detected using methods well known in the art.

Also herein described in the disclosure is a method, typically an *in vitro* or *ex vivo* method, of selecting an appropriate chemotherapeutic treatment of a cancer for a subject having a cancer, typically a renal cancer, more specifically a renal cell carcinoma (RCC), in particular a clear cell renal cell carcinoma (ccRCC) or metastatic ccRCC, which method comprises a step a) of determining, in a biological sample of said subject, the expression level(s) of CXCL5 and/or CXCL7 and a step b) of comparing said CXCL5 and/or CXCL7 level(s) to (CXCL5 and/or CXCL7) reference expression level(s), i) a CXCL5 and/or CXCL7 expression level(s) identical to or below the (CXCL5 and/or CXCL7) reference expression level(s) being the indication that the chemotherapeutic treatment, in particular an inhibitor of a tyrosine kinase receptor or an antibody selected from nivolumab, atezolizumab and avelumab, will not be efficient, in particular will not be efficient alone, against cancer in the subject, and a step c) of selecting an appropriate, preferably optimal, chemotherapeutic treatment of the cancer in the subject; and, on the contrary, ii) CXCL5 and/or CXCL7 expression level(s) above the (CXCL5 and/or CXCL7) reference expression level(s) being the indication that the chemotherapeutic treatment, in particular an inhibitor of a tyrosine kinase receptor or an antibody selected from nivolumab, atezolizumab and avelumab, will be efficient in the subject, said chemotherapeutic treatment being an appropriate chemotherapeutic treatment of the cancer.

A particular method according to the disclosure of selecting, for a subject having a cancer, in particular a renal cancer as herein defined, a cancer treatment or chemotherapy, in particular a treatment comprising an inhibitor of a tyrosine kinase receptor, or an antibody selected from nivolumab, atezolizumab and avelumab, efficient against cancer in the subject, wherein the method comprises a step a) of determining the CXCL5 and/or CXCL7 expression level(s) in a biological sample of the subject having a cancer before any administration to the subject of a cancer treatment, and a step b) of comparing said CXCL5 and/or CXCL7 expression level(s) to (CXCL5 and/or CXCL7) reference expression level(s), CXCL5 and/or CXCL7 expression level(s) identical to the (CXCL5 and/or CXCL7) reference expression level(s) or below the (CXCL5 and/or CXCL7) reference expression level(s), being the indication that the cancer treatment, in particular an inhibitor of a tyrosine kinase receptor or an antibody selected from nivolumab, atezolizumab and avelumab, will not be efficient, or will not be efficient alone, in the subject, and a step c) of selecting an appropriate, preferably optimal, treatment of cancer in the subject, for example combining said cancer treatment, in particular inhibitor of a tyrosine kinase receptor or antibody, with an additional compound such as typically a CXCL5 antibody; and, on the contrary, CXCL5 and/or CXCL7 expression level(s) above the (CXCL5 and/or CXCL7) reference expression level(s) being the indication that the cancer treatment, in particular the inhibitor of a tyrosine kinase receptor or antibody, will be efficient alone against cancer in the subject. Another particular method according to the invention of selecting, for a subject having a cancer, in particular a renal cancer as herein defined, a cancer treatment or chemotherapy, in particular a treatment comprising an inhibitor of a tyrosine kinase receptor, or an antibody selected from nivolumab, atezolizumab and avelumab, efficient against cancer in the subject, comprises a step a) of determining, in a biological sample of said subject, the protein expression level(s) of CXCL5 and in addition the protein expression level of CXCL7, and a step b) of comparing said levels to CXCL5 and CXCL7 reference protein expression levels, a CXCL5 protein expression level identical to the CXCL5 reference protein expression level or below the CXCL5 reference protein expression level together with a CXCL7 protein expression level identical to the CXCL7 reference protein expression level or below the CXCL7 reference protein expression level being the indication that the cancer treatment, in particular a treatment comprising an inhibitor of a tyrosine kinase receptor, or an antibody selected from nivolumab, atezolizumab and avelumab, will not be efficient, or will not be efficient alone, in the subject, and a step c) of selecting an appropriate treatment of cancer in the subject combining said cancer treatment, in particular an inhibitor of a tyrosine kinase receptor or an antibody selected from nivolumab, atezolizumab and avelumab, with an additional compound such as typically a CXCL5 and/or CXCL7 antibody; and, on the contrary, a CXCL5 protein expression level above the CXCL5 reference protein expression level together with a CXCL7 protein expression level above the CXCL7 reference protein expression level being the indication that the cancer treatment, in particular the inhibitor of a tyrosine kinase receptor or the antibody selected from nivolumab, atezolizumab and avelumab, will be efficient alone against cancer in the subject.

In another aspect of the disclosure, the appropriate treatment of cancer as determined in the context of the previously described methods further comprises the administration of CXCL5 and/or CXCL7 antibody(ies), or of an antibody directed against both CXCL5 and CXCL7, for example an antibody directed against CXCL1, CXCL5, CXCL7 and CXCL8 such as antibody 12A10-13 or antibody 35B11-8 (described in WO2014/184384). This(ese) antibody(ies) as well as any antibodies comprising CDR sequences of antibodies 12A10-13 and/or 35B11-8 (selected from SEQ ID NO: 3-14 and any combination thereof) or humanized versions thereof can be used as compensatory molecule(s) allowing the anti-cancer treatment to be effective in the subj ect.

The appropriate treatment of cancer as determined in the context of the previously described methods may, according to a distinct aspect, comprises the administration of an inhibitor of a CXCL5 and/or CXCL7 receptor(s) (CXCR1 and CXCR2) such as SB225002 (Grepin *et al.* 2014), danirixin (GSK1325756 - Miller *et al.*) or CXCR2 SM (Steele *et al*.).

In a further distinct aspect of the disclosure the appropriate treatment of cancer does not involve the cancer treatment, in particular the inhibitor of a tyrosine kinase receptor or antibody, which has been identified as inefficient alone in the subject thanks to the herein above described methods of the disclosure. In this aspect, the appropriate treatment of cancer will preferably be a distinct cancer treatment ("alternative treatment"), typically selected from a distinct cytokine or anti-angiogenic drug, preferably a distinct inhibitor of a tyrosine kinase receptor or mTOR inhibitor, or antibody, as herein described.

In a particular example, when sunitinib has been identified thanks to present disclosure as inefficient alone in the subject, the appropriate treatment of cancer used as alternative treatment will be an inhibitor of a tyrosine kinase receptor as herein described (typically selected from axitinib, pazopanib, sorafenib and a combination thereof), preferably axitinib. This initially selected inhibitor of a tyrosine kinase receptor used in an alternative treatment can further be combined to a mTOR inhibitor as herein described (typically selected from everolimus, temsirolimus and a combination thereof), preferably everolimus, and/or to a further distinct inhibitor of a tyrosine kinase receptor, preferably sorafenib. In a particular embodiment, the mTOR inhibitor and/or further distinct inhibitor of a tyrosine kinase receptor, and the initially selected inhibitor of a tyrosine kinase receptor are used successively, the initially selected inhibitor of a tyrosine kinase receptor being used first and the mTOR inhibitor and/or further distinct inhibitor of a tyrosine kinase receptor being used after said initially selected inhibitor of a tyrosine kinase receptor.

In another particular example, when sunitinib has been identified thanks to present disclosure as inefficient alone in the subject, the appropriate treatment of cancer used as alternative treatment will be a mTOR inhibitor as herein described (typically selected from everolimus, temsirolimus and a combination thereof), preferably everolimus. This initially selected mTOR inhibitor used in an alternative treatment can further be combined to at least one inhibitor of a tyrosine kinase receptor as herein described (typically selected from axitinib, pazopanib, sorafenib and a combination thereof), preferably axitinib alone, or a combination of axitinib and sorafenib, sorafenib and sunitinib, or axitinib, sorafenib and sunitinib which may be used simultaneously or successively. When used in combination, axitinib is preferably used first, and sorafenib, and optionally sunitinib, is/are used after (in this order).

In a further example, when sunitinib has been identified thanks to present disclosure as inefficient alone in the subject, the appropriate treatment of cancer used as alternative and/or combined treatment will be a monoclonal antibody selected from nivolumab, atezolizumab and avelumab.

In the methods herein described in the disclosure of assessing the sensitivity of a subject having a tumor to a chemotherapy as well as in the methods herein described of selecting an appropriate chemotherapeutic treatment, any classical method known by the skilled person of determining the presence or measuring the expression level of a compound of interest, such as typically ELISA and radioimmunoassay can be used.

In some aspect the disclosure relates to a method for monitoring the treatment of a subject suffering from a cancer, typically a renal cancer, comprising i) determining the level(s) of CXCL5 and/or CXCL7 in a sample obtained from the subject before the treatment or during the treatment, ii) determining the level(s) of CXCL5 and/or CXCL7 in a sample obtained from the subject after the treatment or later during the treatment, iii) comparing the level(s) determined at step i) with the level(s) determined at step ii), and iv) concluding that treatment is effective when the level(s) determined at step ii) is (are) above the level(s) determined at step i), or concluding that the treatment is not effective, or is not efficient enough, when the level(s) determined at step ii) is (are) identical to the level(s) determined at step i) or is (are) below the level(s) determined at step i).

When the treatment is identified as not effective or not efficient enough, a method including a step, as herein above described, of selecting an appropriate, preferably optimal, alternative therapeutic treatment of cancer will be advantageously performed.

Methods of screening for candidate therapeutic agents for preventing or treating cancer are also included as part of the disclosure. The method is typically a method which is performed *in vitro* or *ex vivo.* When performed *in vitro* or *ex vivo,* it can be performed for example on a sample from a subject who has been administered with a test compound.

A method herein described in the disclosure is typically a method for screening or identifying a compound suitable for improving the treatment of a cancer in a subject having a tumor, said method comprising determining the ability of a test compound to modify the expression of CXCL5 and/or of CXCL7, or compensate an abnormal (typically absent or insufficient) expression thereof.

In particular, the present disclosure relates to a method of screening for candidate therapeutic agents for a cancer as herein described comprising i) providing a plurality of candidate compounds, ii) bringing the candidate compounds into contact with cancer cell lines, or cancer cells from the subject to be treated (Grépin R., Plos one 2014), expressing CXCL5 and/or CXCL7, in presence of an agent that modulates the expression of CXCL5 and/or of CXCL7, iii) determining the level(s) of CXCL5 and/or of CXCL7 expressed by the cancer cell lines or cancer cells from the subject to be treated, iv) comparing the level(s) determined at step iii) with the level(s) determined in the absence of the candidate compounds, and v) and positively selecting the candidate compounds when the level(s) determined at step iii) is/are decreased when compared to the level(s) determined in the absence of the candidate compounds.

The candidate compound may be a natural or synthetic compound.

Typically, the candidate compound is selected from a cytokine and an anti-angiogenic drug. The anti-angiogenic drug may be for example a tyrosine kinase inhibitor, a serine kinase inhibitor (in particular a mTOR inhibitor), or a threonine kinase inhibitor.

The candidate compound can be any compound tested for its efficiency in the treatment of a carcinoma such as a renal cancer, in particular ccRCC. Volasertib and foretinib are examples of compounds undergoing examination for their efficiency in the treatment of ccRCC in the context of a clinical assay.

The candidate compound according to the disclosure may be selected from a library of compounds previously synthesised, or a library of compounds for which the structure is determined in a database, or from a library of compounds that have been synthesised *de novo.*

In particular, the candidate compound according to the disclosure may be an antibody. The antibodies of the disclosure can for instance be produced by any hybridoma liable to be formed according to classical methods from splenic cells of an animal, particularly of a mouse or rat immunized against an antigenic sequence of interest. The antibodies according to this example of the disclosure may be humanized versions of the mouse antibodies made by means of recombinant DNA technology, departing from the mouse and/or human genomic DNA sequences coding for H and L chains or from cDNA clones coding for H and L chains. Alternatively, the antibodies may be human antibodies. Such human antibodies are prepared, for instance, by means of human peripheral blood lymphocytes (PBL) repopulation of severe combined immune deficiency (SCID) mice or by using transgenic non-human animals capable of producing human antibodies. Also fragments derived from these antibodies such as Fab, F (ab)'2 ands ("single chain variable fragment"), providing they have retained the original binding properties, form part of the present disclosure. Such fragments are commonly generated by, for instance, enzymatic digestion of the antibodies with papain, pepsin, or other proteases. It is well known to the person skilled in the art that monoclonal antibodies or fragments thereof, can be modified for various uses. An appropriate label of the enzymatic, fluorescent, or radioactive type can label the antibodies involved in the disclosure. A typical antibody is an antibody newly identified as a CXCL5 and/or CXCL7 antibody.

In some cases, a candidate therapeutic agent has been identified and further testing may be required. In some embodiments the further testing is to evaluate a candidate therapeutic agent (or an agent that has been confirmed to be therapeutic) for quality control and/or safety concerns. In some embodiments, methods of the disclosure include a method of assaying a therapeutic agent (or candidate therapeutic agent) for efficacy against cancer, typically against a renal cancer, in a relevant animal model.

Also herein described in the disclosure is a method of assessing whether a subject having a renal cancer is at risk of developing cancer metastasis, wherein the method comprises a step of determining the CXCL7 expression level in a biological sample of the subject as herein described, typically in a plasma sample, and a step of comparing said CXCL7 expression level to a CXCL7 reference expression level, a CXCL7 expression level above the reference expression level being the indication that the subject is at risk of developing cancer metastasis or already has cancer metastasis, and a CXCL7 expression level identical to or below the reference expression level being the indication that the subject is not at risk of developing cancer metastasis.

A subject identified as at risk of developing cancer metastasis thanks to the previous method is typically a subject who should be treated with a chemotherapy as herein described, typically a tyrosine kinase inhibitor, preferably sunitinib, or an antibody selected from nivolumab, atezolizumab and avelumab.

A subject identified as not at risk of developing cancer metastasis thanks to the previous method is typically a subject who should not be treated with a chemotherapy as herein described.

The present disclosure also includes kits for assessing or monitoring the sensitivity or resistance of a subject having a cancer, for example a renal cancer, to a chemotherapy. In a particular embodiment, the kit comprises detection means, possibly in suitable container means, selected from the group consisting of at least one CXCL5 polypeptide binding agent (typically an antibody specific to CXCL5 such as the CXCL5 specific antibody generated by R&D Systems reference DY254) and/or at least one CXCL7 polypeptide binding agent (typically an antibody specific to CXCL7 such as one of the CXCL7 specific antibodies generated by Peprotech, for example Peprotech' reference 900-K40); optionally a molecule allowing the binding agent(s) (typically antibody(ies)) detection; and, optionally, a leaflet providing the CXCL5 and/or CXCL7 reference expression level(s) in a biological sample from a control or reference population.

In further aspects of the disclosure, the binding agent is labeled or a detection agent is included in the kit. It is contemplated that the kit may include CXCL5 and/or CXCL7 polypeptide binding agent(s) attached to a non-reacting solid support, such as a tissue culture dish or a plate with multiple wells. It is further contemplated that such a kit includes at least one, typically two (one for each of CXCL5 and CXCL7 polypeptide binding agents), detectable agent(s) in certain examples of the disclosure. In some examples, the disclosure concerns kits for carrying out a method of the disclosure comprising, in suitable container means: (a) an agent that specifically recognizes all or part of a CXCL5 or CXCL7 polypeptide, preferably an agent that specifically recognizes all or part of a CXCL5 polypeptide and an agent that specifically recognizes all or part of a CXCL7 polypeptide; and, (b) positive control(s) that can be used to determine whether the agent is capable of specifically recognizing all or part of a CXCL5 or CXCL7 polypeptide. The kit may also include other reagents that allow visualization or other detection of the CXCL5 and/or CXCL7 polypeptide, such as reagents for colorimetric or enzymatic assays.

The present disclosure further relates to the use of a kit as herein described i) for assessing or monitoring the sensitivity or resistance of a subject having a cancer, in particular a renal cancer, to a chemotherapy, in particular to an inhibitor of a tyrosine kinase receptor selected from sunitinib, axitinib, pazopanib and sorafenib, or to an antibody selected from nivolumab, atezolizumab and avelumab, and/or ii) for determining the potential toxicity of said chemotherapy in a subject having a cancer, in particular a renal cancer.

Throughout this application, various references describe the state of the art to which this disclosure pertains.

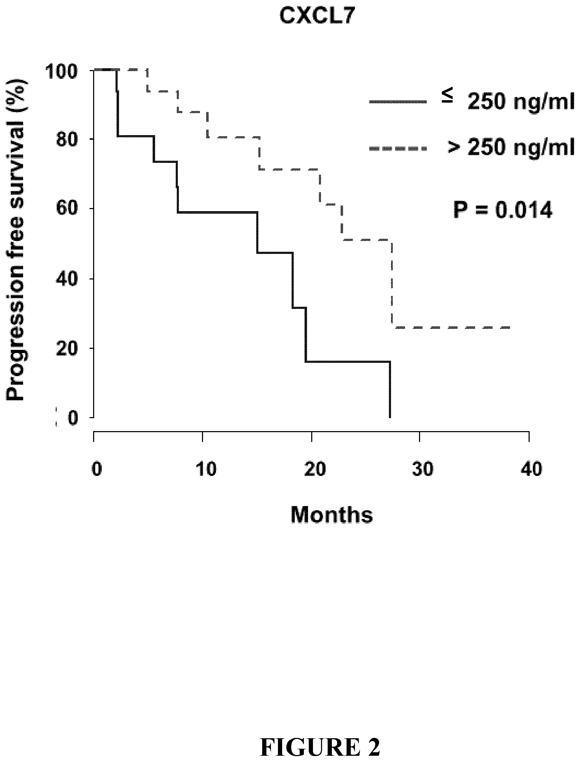

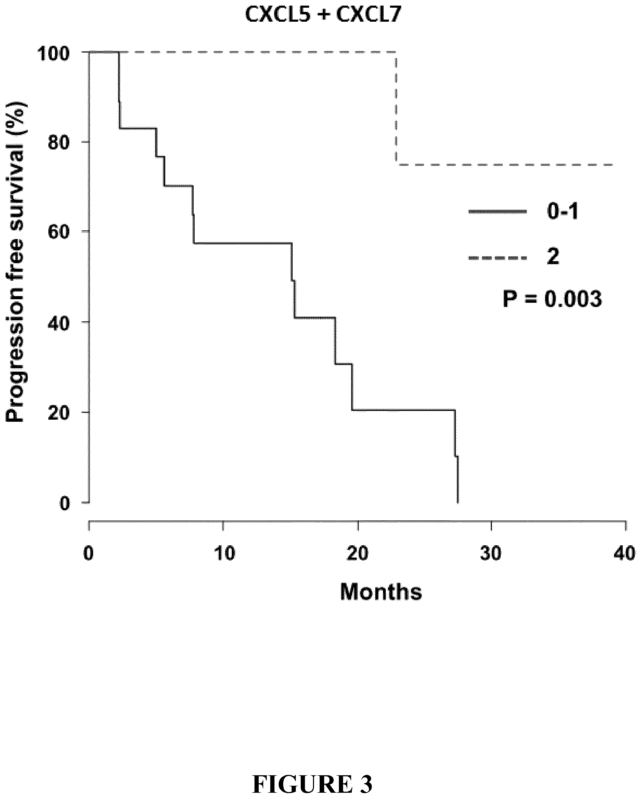

Other characteristics and advantages of the invention are given in the following experimental section (with reference to figures 1 to 9), which should be regarded as illustrative and not limiting the scope of the present application.

### FIGURES

**Figure 1****.** The Kaplan-Meier method was used to produce progression-free survival curves and the log-rank test was employed to determine statistical differences between the survival curves. The CXCL5 cut-off point (100 pg/ml) for PFS survival was determined using spline curves analysis.
**Figure 2****.** The Kaplan-Meier method was used to produce progression-free survival curves and the log-rank test was employed to determine statistical differences between the survival curves. The CXCL7 cut-off point (250 ng/ml) for PFS survival was determined using spline curves analysis.
**Figure 3****.** The Kaplan-Meier method was used to produce progression-free survival curves and the log-rank test was employed to determine statistical differences between the survival curves. The patients were stratified according to their CXCL5 and CXCL7 levels above or below the reference values determined above. Patients 0 (two cytokines below the reference); patients 1 (one of the cytokine below the reference); patients 2 (two cytokines above the reference value).
**Figure 4****.** The Kaplan-Meier method was used for analysis of progression-free survival (A) and overall survival (B) of patients with ccRCC treated with sunitinib. Progression-free survival was calculated from patient subgroups with plasmatic level of CXCL7 at the diagnosis that were less or greater than a cut off value of 250 ng/ml, for SUVEGIL trial (prospective analysis). Statistical significance (p value) and the time of the median disease free are indicated.

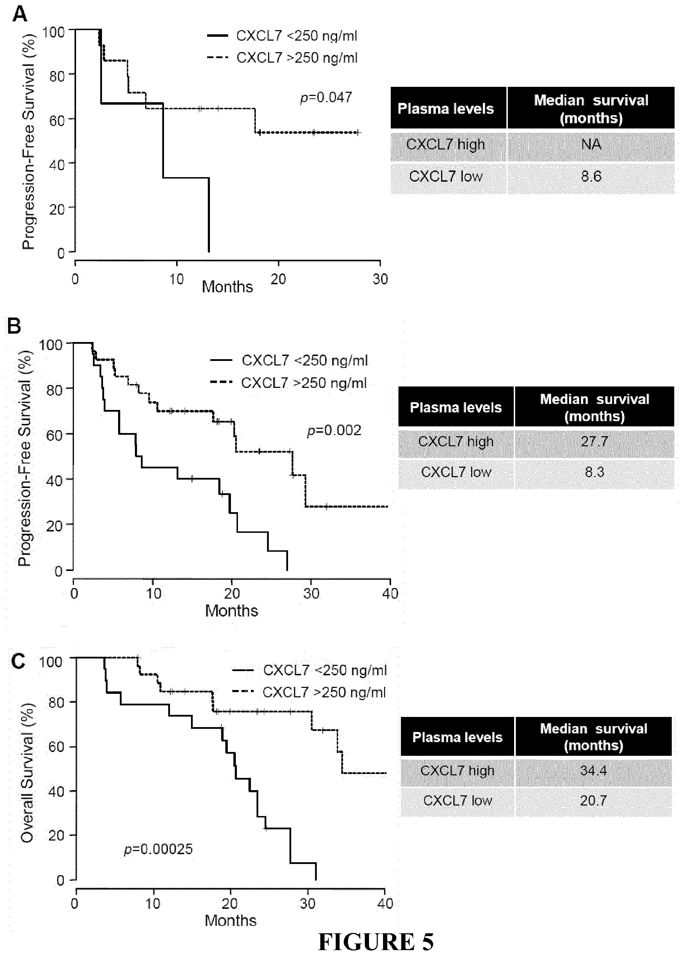

**Figure 5****.** The Kaplan-Meier method was used for analysis of progression-free survival of patients with ccRCC treated with sunitinib. Progression-free survival was calculated from patient subgroups with plasmatic level for CXCL7 at the diagnosis that were less or greater than a cut off value of 250 ng/ml for TORAVA trial (retrospective analysis) (A) and progression-free survival (B) and overall survival (C) was calculated from patient subgroups with plasmatic level of CXCL7 at the diagnosis that were less or greater than a cut off value of 250 ng/ml for combination of SUVEGIL and TORAVA trials. Statistical significance (p value) and the time of the median disease free are indicated.
**Figure 6****.** The Kaplan-Meier method was used for analysis of progression-free survival of patient with ccRCC treated with bevacizumab and temsirolimus (A) or bevacizumab and interferon (B). Progression-free survival was calculated from patient subgroups with plasmatic level of CXCL5 at the diagnosis that were less or greater than a cut off value of 0.1 ng/ml for TORAVA trial. Statistical significance (p value) and the time of the median disease free are indicated.
**Figure 7****.** The Kaplan-Meier method was used for analysis of progression-free survival of patient with ccRCC treated with bevacizumab and temsirolimus (A) or bevacizumab and interferon (B). Progression-free survival was calculated from patient subgroups with plasmatic level for CXCL7 at the diagnosis that were less or greater than a cut off value of 250 ng/ml for TORAVA trial. Statistical significance (p value) and the time of the median disease free are indicated.
**Figure 8****.** CXCL5 plasmatic levels of healthy donor compared to ccRCC patients.
**Figure 9****.** CXCL7 plasmatic levels of healthy donor compared to ccRCC patients (A) and of healthy donor compared to sunitinib responsive patients and patients that relapse on a sunitinib treatment (B).

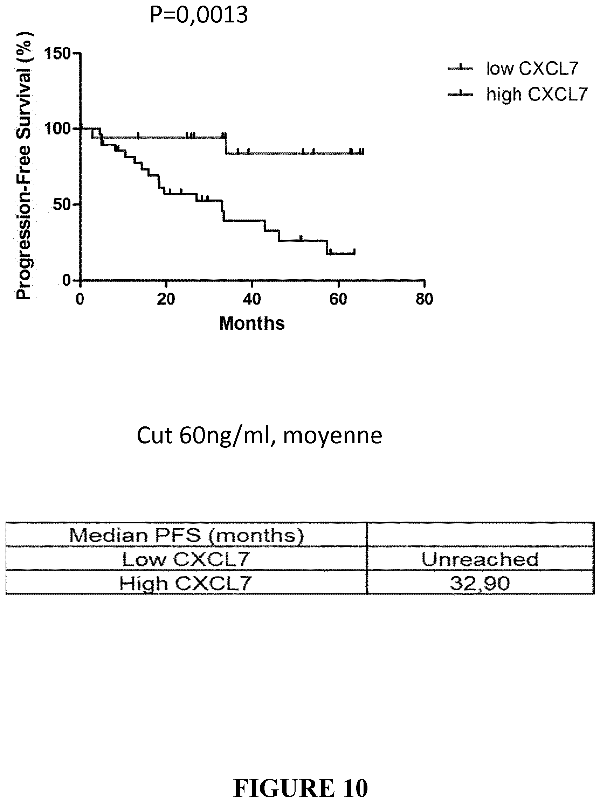

**Figure 10****.** Survival of non-metastatic patients stratified on CXCL7 levels The predictive role of CXCL7 was assessed in 46 untreated patients.
   Patients with CXCL7 plasma levels below 60 ng/ml had a longer PFS (> 80 months) compared to patients with plasma levels above 60 ng/ml (median 32.9 months, p = 0.0013).

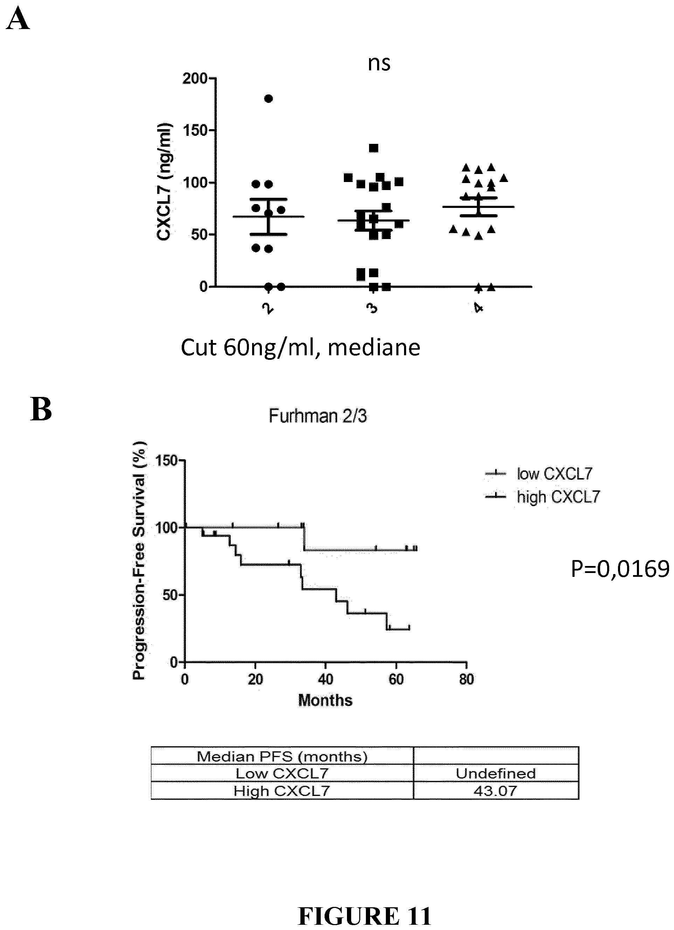

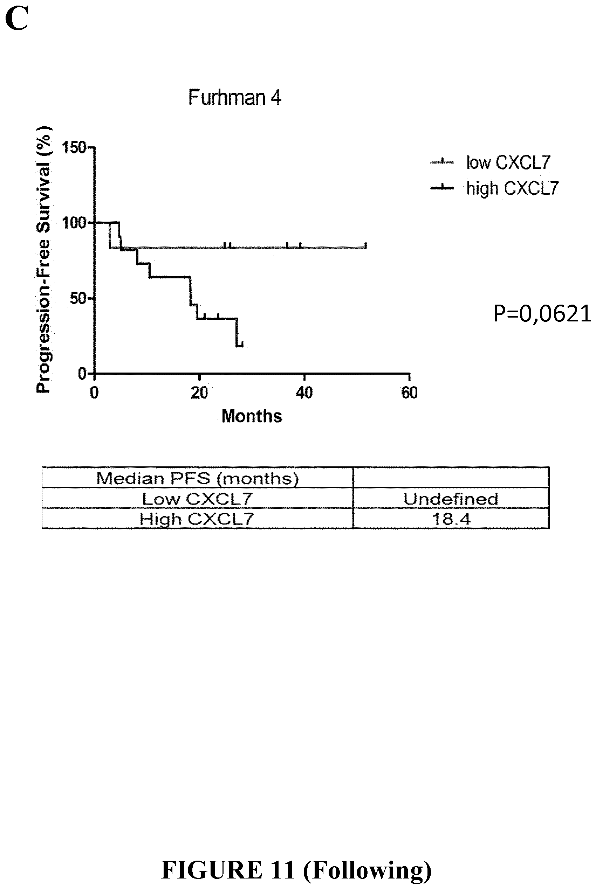

**Figure 11****.** Independency of clinical parameters; Fuhrman grade
   A) CXCL7 levels were tested in the plasmas of patients whose tumors are of different Fuhrman grades. No statistical differences were observed (ns).
   B) The PFS of patients with Fuhrman grades 2/3 were tested according to their plasmatic CXCL7. Patients with CXCL7 plasma levels below 60 ng/ml ("low") had a longer PFS (> 80 months) compared to patients with plasma levels above 60 ng/ml ("high") (median 43.07 months, p = 0.0169).
   C) The PFS of patients with Fuhrman grade 4 were tested according to their plasmatic CXCL7. Patients with CXCL7 plasma levels below 60 ng/ml ("low") had a longer PFS (> 80 months) compared to patients with plasma levels above 60 ng/ml ("high") (median 18.04 months, p = 0.0621). These results showed a trend for longer PFS.

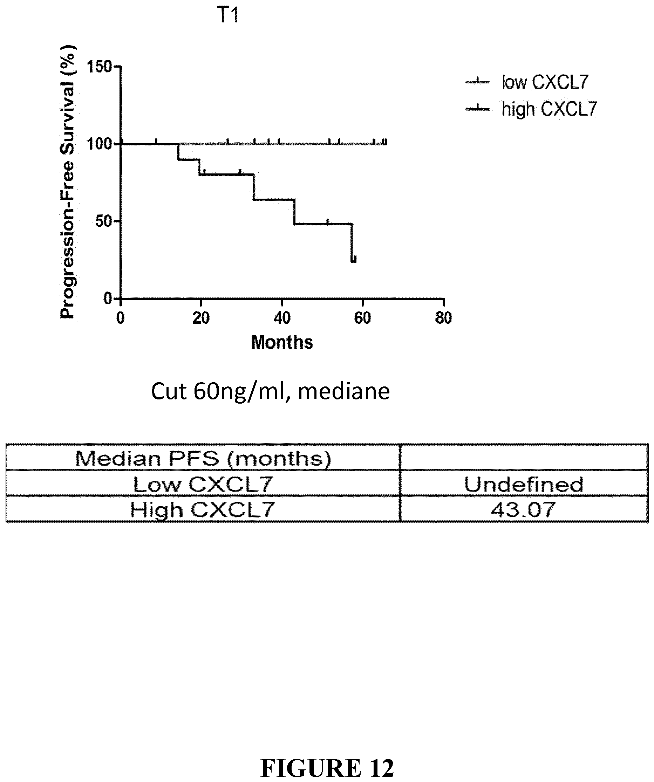

**Figure 12****.** Independency versus clinical parameters; Size (T1 < 7 cm)
   CXCL7 levels were tested in the plasmas of patients whose tumors have a small/intermediate sizes (< 7 cm). The PFS of patients were tested according to their plasmatic CXCL7. Patients with CXCL7 plasma levels below 60 ng/ml (« low ») had a longer PFS (> 80 months) compared to patients with plasma levels above 60 ng/ml ("high") (median 43.07 months, p = 0.0131).

### EXPERIMENTAL PART

### EXAMPLE 1 - Discovery of predictive biomarkers of sunitinib response in clear cell renal cell carcinoma (ccRCC).

### Patients/Methodology

Patients are from a cohort included in the clinical trial SUVEGIL8, a multicenter clinical trial having for promoter the Centre Antoine Lacassagne and as recruiters centers: the Clinique de Mougins, the Clinique Saint Jean du Languedoc (Toulouse), the Institut Daniel Hollard (Grenoble), and Assistance Publique Hôpitaux de Marseille (Centre hospitalier de la Timone).

**Table 1: Description of the population**

| | **TOTAL** |
|---|---|
| **TOTAL** | 37 |

| **AGE** | |
|---|---|
| Mean (SD) | 63.6 (10.3) |

| **SEX** | |
|---|---|
| F | 8 (21.6) |
| M | 29 (78.4) |

| **Fuhrman Grade** | |
|---|---|
| 1 | 1 (3.2) |
| 2 | 8 (25.8) |
| 3 | 17 (54.8) |
| 4 | 5 (16.1) |

| **Fuhrman Grade (categories)** | |
|---|---|
| 1-2 | 9 (29) |
| 3-4 | 22 (71) |

| **pT (size)** | |
|---|---|
| 1 | 6 (17.6) |
| 2 | 7 (20.6) |
| 3 | 14 (41.2) |
| 4 | 7 (20.6) |

| **pN (lymph node invasion)** | |
|---|---|
| 0 | 13 (38.2) |
| 1 | 1 (2.9) |
| 2 | 3 (8.8) |
| X | 17 (50) |

| **pM (metastatic status)** | |
|---|---|
| 0 | 13 (38.2) |
| 1 | 8 (23.5) |
| X | 13 (38.2) |

| **Stage** | |
|---|---|
| Stage I | 3 (17.6) |
| Stage II | 1 (5.9) |
| Stage III | 5 (29.4) |
| Stage IV | 8 (47.1) |

| **Relative dose intensity** | |
|---|---|
| =/<80% | 13 (35.1) |
| >80% | 24 (64.9) |

| **Number of metastatic sites** | |
|---|---|
| =/>3 | 7 (18.9) |
| 1 | 18 (48.6) |
| 2 | 12 (32.4) |

| **Time from** d**iagnosis of the primary tumor to treatment with sunitinib** | |
|---|---|
| < 1 year | 15 (45.5) |
| =/> 1 year | 18 (54.5) |

| **Score** | |
|---|---|
| 0-1 | 19 (67.9) |
| 2 | 9 (32.1) |

| | |
|---|---|
| Score: 0-1: no or high level of a single cytokine - 2: high levels of two cytokines | |

### Methodology

### . Statistics

The reference levels of each cytokine were determined from the cut-off obtained with the spline curves of the hazard ratio for progression-free survival.

### . Results

The results of the statistical study show that levels of CXCL5 and CXCL7 at diagnosis are predictive of response to treatment with sunitinib.

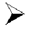 **CXCL5 (invention)**

The study identifies a CXCL5 reference expression level (threshold) of 100 pg/ml plasma, a plasma level lower than levels detected in heathly donors (median 565 range: 51-2367 pg/ml) (Tacke *et al.,* 2011). Patients with plasma levels below 100 pg/ml, which corresponds to the median of observed values (range 0-100 pg/ml) had a poor outcome compared to patients whose plasma levels are above 100 pg/ml (range : 100-821.8 pg/ml).

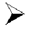 **CXCL7 (disclosure)**

The study identifies a CXCL7 reference expression level (threshold) of 250 ng/ml plasma, a plasma level equivalent to levels detected in heathly donors (mean 183 ± 321 ng/ml) (Glenister *et al.,* 2008; Ayache *et al.,* 2006). Patients with plasma levels below 250 ng/ml, which corresponds to the median of observed values (range 139.2-250 ng/ml) had a poor outcome compared to patients whose plasma levels are above 250 ng/ml (range : 250-485.2 ng/ml).

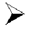 **Combination of two parameters (CXCL5 and CXCL7) (invention)**

The statistical study focused on the cumulative effect of the two parameters on the predictability of response to sunitinib. Thus, two populations of patients were stratified: patients with 0 and 1 parameter above or below the previously determined threshold, and patients with both parameters above or below the threshold. Thus, patients with plasma levels of CXCL5 above 100 pg/ml and with plasma levels of CXCL7 above 250 ng/ml are sensitive to sunitinib for a longer time.

### Conclusions

Levels close to the normal of cytokine CCL5 and CXCL7 post-surgery and just before starting treatment are predictive of response to sunitinib.

### EXAMPLE 2 - Predictive biomarkers of sunitinib response in clear cell renal cell carcinoma (ccRCC).

### PATIENTS AND METHODS

### Patients

Eligible patients were at least 18 years of age and had metastatic ccRCC histologically confirmed, with the presence of measurable disease according to Response Evaluation Criteria in Solid Tumors (RECIST) v1.1. All patients gave written informed consent. The SUVEGIL trial (NCT00943839) was a multicenter prospective single-arm study. The TORAVA trial (NCT00619268) was used as a validation cohort. Patient characteristics and results have been previously described (Negrier *et al.,* 2011).

### Efficacy and safety

The primary end point of the study was to evaluate the correlation between plasmatic levels of CXCL5 and CXCL7 and the overall response rate (ORR). The response was evaluated by investigators' assessment.

The secondary end points were to evaluate the correlation between plasmatic levels of CXCL5 and/or CXCL7 and overall survival (OS) and progression-free survival (PFS), censored at last follow-up for those still alive or who have not progressed.

Safety was assessed at the end of each cycle (after four weeks of sunitinib) by documentation of adverse events and physical examination. Adverse events were graded with the use of the Common Terminology Criteria for Adverse Events of the National Cancer Institute, version 3.0.

### Biochemical Analysis

Blood samples were centrifuged and the plasmas were collected and conserved at -80°C. Plasmatic levels of CXCL5 and CXCL7 were determined by ELISA using R&D (reference DY254) and Peprotech kits (reference 900-K40) respectively. Pure plasma or a 1/5 dilution was used for CXCL5 detection and a 1/100 dilution was used to detect CXCL7.

### Statistical analysis

With 61 patients, it was calculated that the study will provide 90% power to demonstrate a 0.4 correlation coefficient between response and chemokine plasmatic levels with a two sided 0.05 fixed alpha risk. Secondary endpoints were PFS and OS.

Spearman's correlation was performed between best response rate and quantitative level of chemokine. The CXCL5 and CXCL7 cut-off points (respectively 0.1 and 250 ng/ml) for PFS and OS were determined using smoothing spline curves analysis (R software, version 3.2.2). Kaplan Meier method was used to produce survival curves and analyses of censored data were performed using Cox models. The predictive cut-off points determined in the SUVEGIL cohort were used for the TORAVA cohort.

### Healthy donor plasma

Plasmas were obtained from healthy donors with informed consent following the Declaration of Helsinki according to recommendations of an independent scientific review board.

### RESULTS

### Patient and pathological parameters

Between March 2009 and October 2013, 37 patients were enrolled in the SUVEGIL trial at five French centers. At initial diagnosis, the median age was 63.6 years. All patients undergo nephrectomy. 13 patients (35%) had regional or distant nodal invasion and 6 patients (16%) had distant lymph node metastases. Eleven (30%) patients had Fuhrman grade 1-2 and 26 (70%) patients had Fuhrman grade 3-4 tumors. The time from diagnosis to apparition of metastasis was inferior to one year for 17 patients (45.9%) and superior to one year for 20 patients (54.1%). 16 (43%) patients had a good MSKCC score and 14 (38%) patients had intermediate or bad MSKCC score. The population characteristics and pathological parameters are summarized in Table 2. Patients' characteristics from the TORAVA trials included in the validation cohort are also described in Table 2.

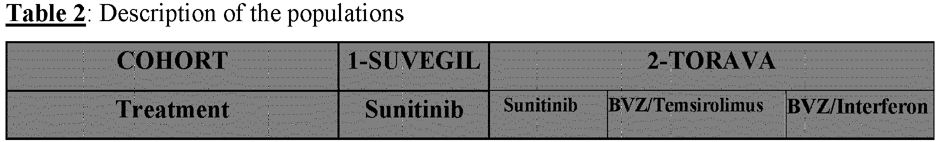

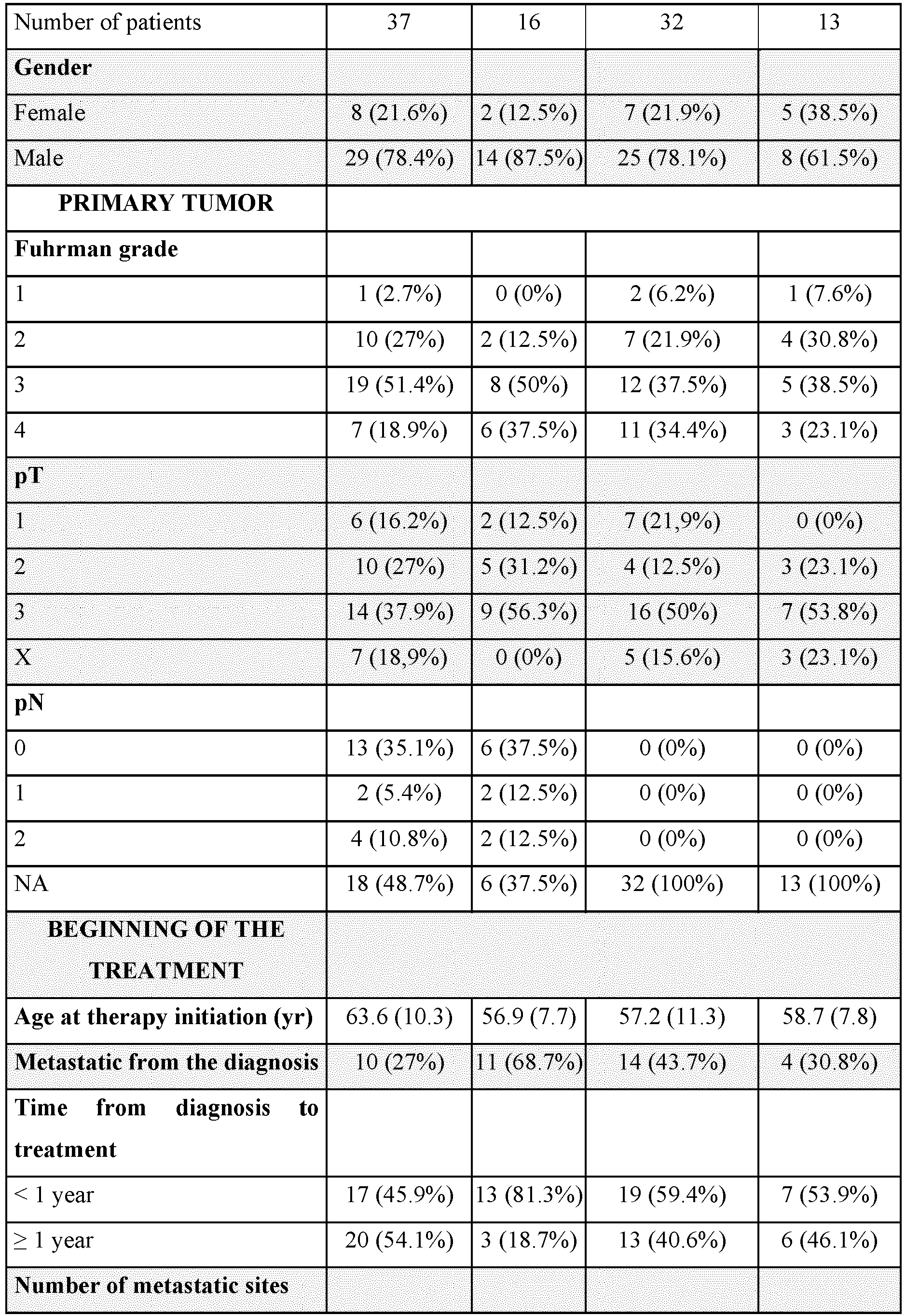

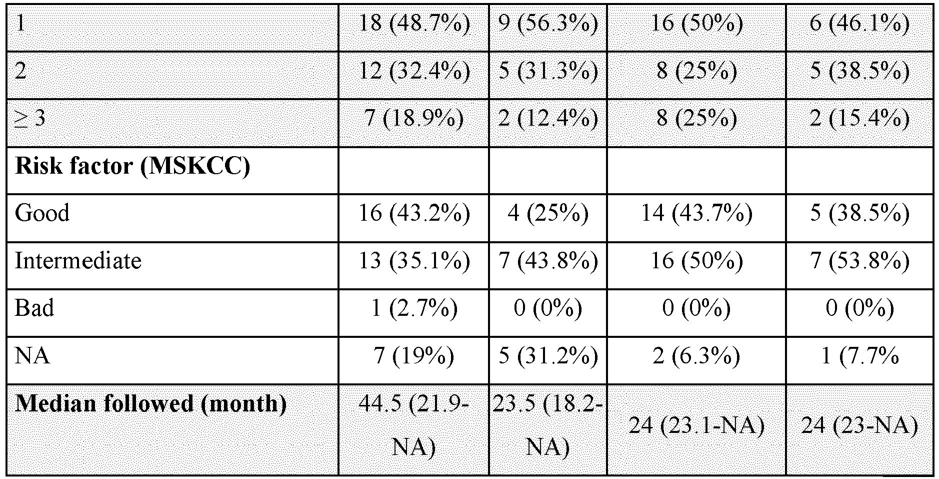

### Survival (PFS and OS) in prospective cohort and correlation to CXCL5/7 plasmatic levels

The median PFS was 20.1 months and the median OS was 24.6 months. The plasmatic level of CXCL5 and CXCL7 were measured at diagnosis and throughout the different cycles of sunitinib treatments. No correlation was observed between disease progression and the variation in cytokine plasmatic levels along time. Only plasmatic levels at diagnosis were correlated to PFS.

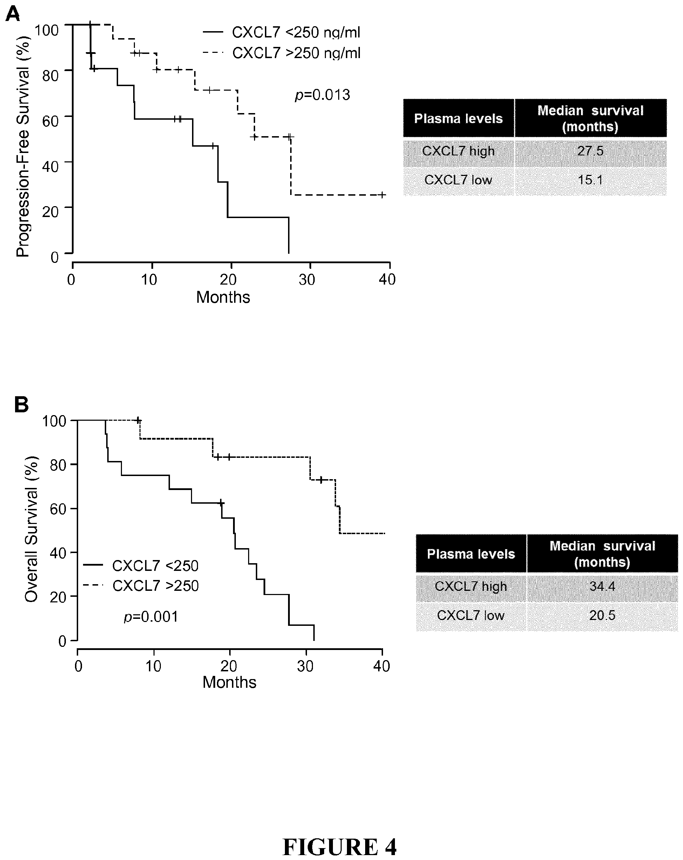

Patients with CXCL7 plasma levels below 250 ng/ml (range 139.2-250 ng/ml) had a shorter PFS (15.1 months) compared to patients with plasma levels above 250 ng/ml (range 250 - 485.2 ng/ml - 27.5 months, p = 0.013) (Fig. 4A).

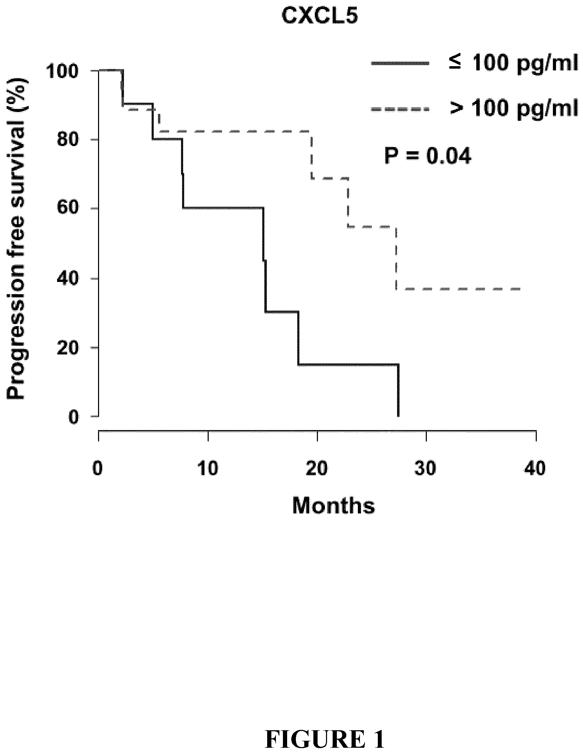

Patients with CXCL5 plasma levels below 0.1 ng/ml (range 0-0.1 ng/ml) also had a poor outcome, with a median PFS of 14.9 months compared to 27 months for patients whose plasmatic levels were above 0.1 ng/ml (range: 0.1-0.82 ng/ml), p = 0.04 (Fig. 1A). The levels of CXCL7 (cut off: 250 ng/ml) was also correlated with OS. Indeed, patients with plasma levels below 250 ng/ml had a lower median OS (20.5 months) compared to patients with plasmatic levels above 250 ng/ml (34.4 months, p = 0.001) (Fig. 4B).

### Survival (PFS and OS) in validation retrospective cohort (sunitinib group) and correlation to CXCL5 and/or CXCL7 plasmatic levels.

The predictive role of CXCL5 and CXCL7 was assessed in 16 patients treated with sunitinib in an independent retrospective cohort (TORAVA trial).

Patients with CXCL7 plasma levels below 250 ng/ml had a shorter PFS (8.6 months) compared to patients with plasma levels above 250 ng/ml (not reached, p = 0.047) (Fig. 5A).

By combining both cohorts, the statistical significance was improved. Indeed, patients with CXCL7 plasma levels below 250 ng/ml had a shorter median PFS (8.3 months) compared to patients with plasma levels above 250 ng/ml (27.7 months, p = 0.002) (Fig. 5B).

Patients with CXCL7 plasma levels below 250 ng/ml had a shorter OS (20.7 months) compared to patients with plasma levels above 250 ng/ml (34.4 months, p = 0.00025) (Fig. 5C).

### Correlation between PFS and OS and CXCL5 and/or CXCL7 plasmatic levels in the retrospective cohort for bevacizumab/temsirolimus or bevacizumab/interferon groups.

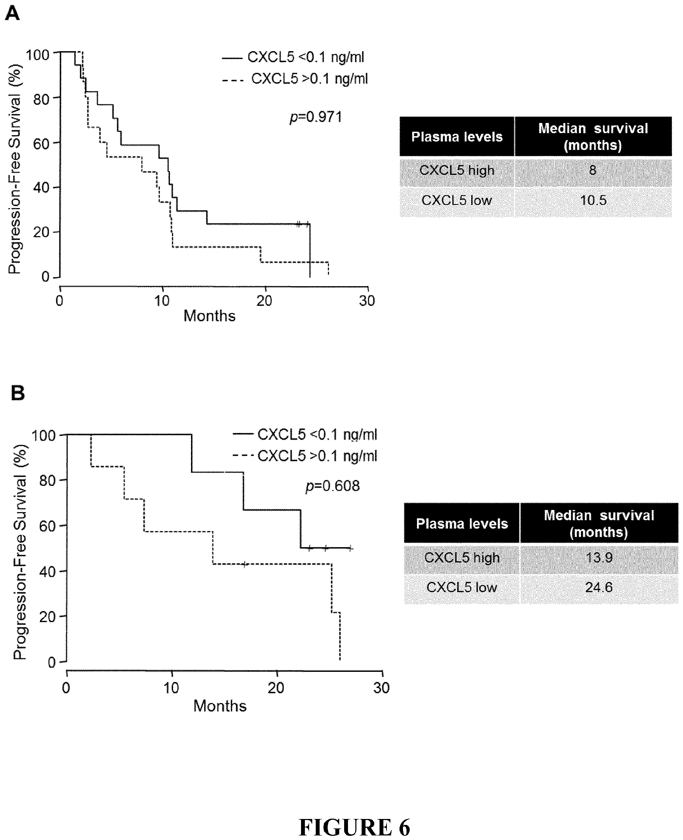

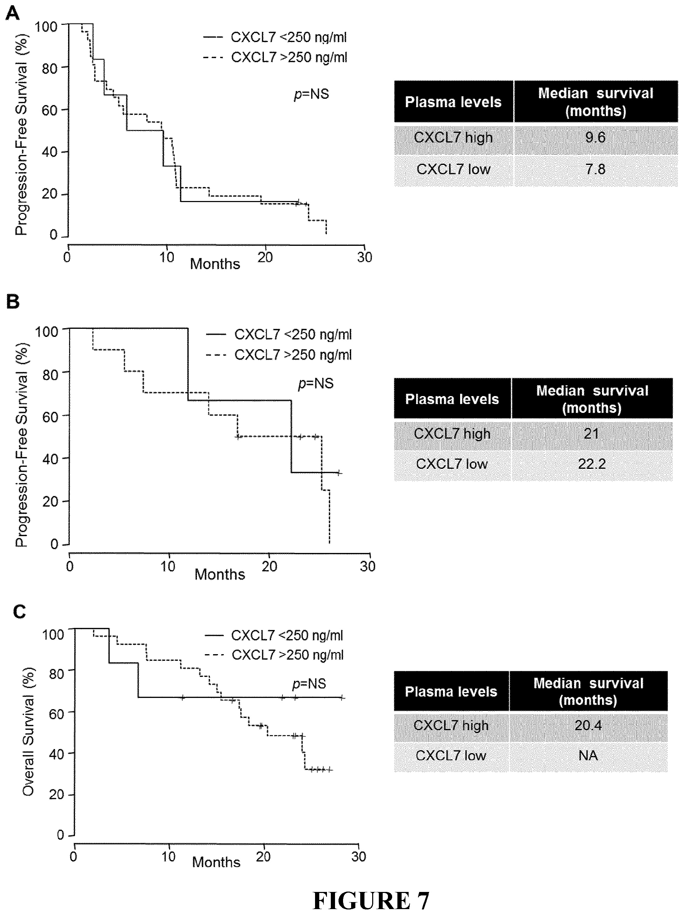

To determine the predictive role of CXCL5/7 for sunitinib efficacy, inventors tested the plasmatic levels of CXCL5/7 in a subset of patients of the TORAVA clinical trial that was treated with bevacizumab/temsirolimus or bevacizumab/interferon alpha. In both subgroups, CXCL5 (CXCL5 < 0.1 ng/ml, median PFS: 10.5 or 24.6 months, CXCL5 > 0.1 ng/ml, median PFS: 8 or 13.9 months, p = 0.971 or 0.608, Fig. 6 A and B) and CXCL7 (CXCL7 < 250 ng/ml, median PFS: 7.8 or 21 months, CXCL5 > 250 ng/ml, median PFS: 9.6 or 22.2 months, p = NS, Fig. 7 A to C) plasmatic levels did not discriminate patients with a long or a short PFS or OS.

### Additional exploraty analyzes

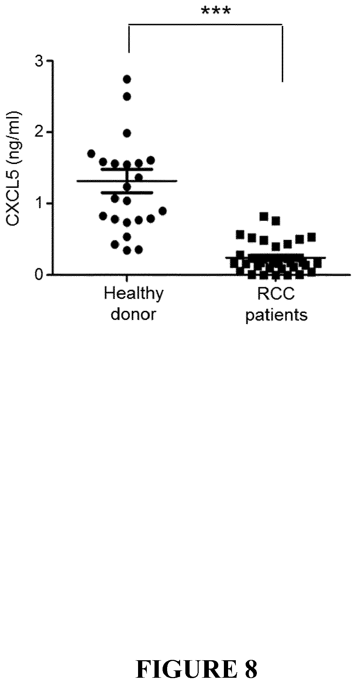

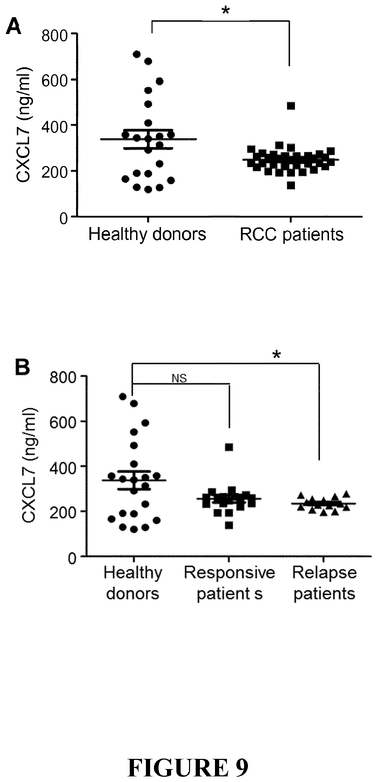

Inventors compared the levels of CXCL5 and CXCL7 in the plasma of 24 healthy donors and 37 ccRCC patients (following surgical removal of the primary tumor). The level of CXCL7 and CXCL5 is lower in ccRCC patients (1.4 fold and 6 fold respectively) (Fig. 9A and Fig. 8A). Moreover, patients that relapse on sunitinib have decreased plasmatic CXCL7 levels compared to responsive patients, whose CXCL7 plasmatic concentrations are not significantly different from healthy donors (Fig. 9B).

### DISCUSSION

Despite the development of several therapies for ccRCC and the increase of PFS, no curative treatment currently exists. Because of the heterogeneity of the initial tumors and their subsequent metastasis (Gerlinger *et al.,* 2014), the response to sunitinib is highly variable. There are two major constraints. The first one is related to the improvement of patient survival. The second is economical and related to the high costs of targeted therapies. In order to reconcile the therapeutic and the economic imperatives, robust predictive markers of treatment efficacy have to be identified. To be manageable in the clinical practices, protocols must be easy to transfer to technical platforms of Hospitals, must be non-invasive and applicable to small blood or urine samples. Inventors have identified such a marker in the frame of a prospective multicenter clinical trial (SUVEGIL), the angiogenic and pro-inflammatory cytokine CXCL7. This was corroborated in another patient cohort highlighting the relevance of inventors' results. Moreover, CXCL7 was not indicative of PFS or OS for patients treated with other drug combinations. These results are indicative of the predictive value of CXCL7 for sunitinib efficacy. Targets being equivalents, we believe that those results can be extrapolated to axitinib, pazopanib and sorafenib. Those results could in addition be extrapolated to nivolumab, atezolizumab and avelumab. Indeed, Liu *et al.* showed a sunitinib-dependent induction of PDL-1 on tumor cells.

### CONCLUSION

Inventors highlighted CXCL5, CXCL7 and a combination thereof as predictive markers of sunitinib efficacy. They established a threshold value for patients for low or high risk of relapse and death in independent prospective cohort and in a retrospective cohort.

### EXAMPLE 3 - Correlation between CXCL7 plasmatic levels and survival in non-metastatic patients (M0)

46 non metastatic patients (M0) were enrolled in the study. The median progression free survival of these patients was 46.3 months (PFS). Of the 46 patients 19 became metastatic after a variable time. The plasmas of these patients were analyzed as described in example 2.

The median value for CXCL7 expression was 60 ng/ml. This threshold was chosen as the cut of value for PFS analysis.

CXCL7 plasmatic level is an independent marker of survival as compared to Fuhrman grade and tumor size.

### REFERENCES

- Ayache S., Panelli MC, Byrne M, et al. Comparison of proteomic profiles of serum, plasma, and modified media supplements used for cell culture and expansion. J Transl Med 2006;4:40.
- Cerami E, Gao J, Dogrusoz U, Gross BE, Sumer SO, Aksoy BA, et al. The cBio cancer genomics portal: an open platform for exploring multidimensional cancer genomics data. Cancer Discov. 2012;2:401-4.
- Escudier B, Bellmunt J, Negrier S, et al. Phase III trial of bevacizumab plus interferon alfa-2a in patients with metastatic renal cell carcinoma (AVOREN): final analysis of overall survival. J Clin Oncol 2010; 28(13):2144-50.- Fu WX, Gong SY, Qian XP, Li Y, Zhu ML, Dong XY, et al. Differential chemotactic potential of mouse platelet basic protein for thymocyte subsets. Cell Mol Life Sci. 2004;61:1935-45.
- - Galkina E, Ley K. Leukocyte influx in atherosclerosis. Curr Drug Targets. 2007;8: 1239-48.
- Galliera E, Corsi MM, Banfi G. Platelet rich plasma therapy: inflammatory molecules involved in tissue healing. J Biol Regul Homeost Agents. 2012;26:35S-42S.
- Gao J, Aksoy BA, Dogrusoz U, Dresdner G, Gross B, Sumer SO, et al. Integrative analysis of complex cancer genomics and clinical profiles using the cBioPortal. Sci Signal. 2013;6:pl1.
- Gerlinger M, Horswell S, Larkin J, Rowan AJ, Salm MP, Varela I, et al. Genomic architecture and evolution of clear cell renal cell carcinomas defined by multiregion sequencing. Nat Genet. 2014;46:225-33.
- Glenister KM, Payne KA, Sparrow RL. Proteomic analysis of supernatant from pooled buffy-coat platelet concentrates throughout 7-day storage. Transfusion 2008; 48(1):99-107.
- Grépin R, Ambrosetti D, Marsaud A, Gastaud L, Amiel J, Pedeutour F, Pages G. The relevance of testing the efficacy of anti-angiogenesis treatments on cells derived from primary tumors: a new method for the personalized treatment of renal cell carcinoma. Mar 27;9(3):e89449. doi: 10.1371/journal.pone.0089449.
- Grépin R, Guyot M, Giuliano S, et al. The CXCL7/CXCR1/2 axis is a key driver in the growth of clear cell renal cell carcinoma. Cancer Res. 2014; 74(3):873-83.
- Grépin R, Guyot M, Jacquin M, et al. Acceleration of clear cell renal cell carcinoma growth in mice following bevacizumab/Avastin treatment: the role of CXCL cytokines. Oncogene 2012; 31(13): 1683-94.
- Karaman S, Detmar M. Mechanisms of lymphatic metastasis. J Clin Invest. 2014; 124:922-8.
- Liu XD, Hoang A, Zhou L, Kalra S, et al. Resistance to antiangiogenic therapy is associated with an immunosuppressive tumor microenvirnment in metastatic renal cell carcinoma. Cancer Immunol res. 2015 Sept. 3(9): 1017-29.
- Miller B., Mistry S., et al. The pharmacokinetics and pharmacodynamics of danirixin (GSK1325756) - selective CXCR2 antagonist - in healthy adult subjects. BMC pharmacology and toxicology (2015) 16:18.
- Motzer RJ, Hutson TE, Tomezak P, et al. Sunitinib versus interferon alfa in metastatic renal-cell carcinoma. N Engl J Med 2007 ; 356(2) : 115-24.
- Motzer RJ, Escudier B, Tomezak P, et al. Axitinib versus sorafenib as second-line treatment for advanced renal cell carcinoma: overall survival analysis and updated results from a randomised phase 3 trial. Lancet Oncol 2013; 14(6):552-62.
- Motzer RJ, Hutson TE, Cell D, et al. Pazopanib versus sunitinib in metastatic renal-cell carcinoma. N Engl J Med 2013; 369(8):722-31.
- Motzer RJ, Escudier B, Oudard S, et al. Efficacy of everolimus in advanced renal cell carcinoma: a doubleblind, randomised, placebo-controlled phase III trial. Lancet 2008; 372(9637):449-56.
- Mulders PF, Brouwers AH, Hulsbergen-van der Kaa, van Lin EN, Osanto S, de Mulder PH; Ned Tijdschr Geneeskd, 'Guideline renal cell carcinoma'; 2008 Feb 16; 152(7): 376-80.
- Negrier S, Gravis G, Perol D, Chevreau C, Delva R, Bay JO, et al. Temsirolimus and bevacizumab, or sunitinib, or interferon alfa and bevacizumab for patients with advanced renal cell carcinoma (TORAVA): a randomised phase 2 trial. Lancet Oncol. 2011;12:673-80.
- Rini BI, Rathmell WK, Godley P., 'Renal cell carcinoma'; 2008 May; Curr Opin Oncol, 20(3): 300-6.
- Steele C. et al. CXCR2 inhibition profoundly suppresses metastases and augments immunotherapy in pancreatic ductal adenocarcinoma. Cancer Cell 29, 832-845.
- Tacke F, Zimmermann HW, Trautwein C, et al. CXCL5 plasma levels decrease in patients with chronic liver disease. J Gastroenterol Hepato 2011; 26(3):523-9.
- von Hundelshausen P, Petersen F, Brandt E. Platelet-derived chemokines in vascular biology. Thromb Haemost. 2007;97:704-13.
- Yu M, Berk R, Kosir MA. CXCL7-Mediated Stimulation of Lymphangiogenic Factors VEGF-C, VEGF-D in Human Breast Cancer Cells. J Oncol. 2010;2010:939407.

## Claims

1. An *in vitro* method of assessing the sensitivity of a subject having a renal cancer to an inhibitor of a tyrosine kinase receptor, for treating renal cancer, which method comprises a step a) of determining, in a biological sample from said subject, the protein expression level of CXCL5, and, when the CXCL5 protein expression level is determined, a step b) of comparing said CXCL5 protein expression level to a CXCL5 reference protein expression level, thereby assessing whether the subject having ccRCC is sensitive or resistant to the inhibitor of a tyrosine kinase receptor, wherein the biological sample is selected from a blood sample, a serum sample, a plasma sample , wherein CXCL5 protein expression level above said reference protein expression level is indicative of a sensitivity of the subject to the inhibitor of a tyrosine kinase receptor.

2. The method according to claim 1, wherein the method further comprises a step a') of determining, in a biological sample from said subject, the protein expression level of CXCL7 and, when the CXCL7 protein expression level is determined, a step b') of comparing said CXCL7 protein expression level to a CXCL7 reference protein expression level.

3. The method according to claim 1 or claim 2, of assessing the sensitivity of a subject having a renal cancer to a chemotherapeutic combination of an inhibitor of a tyrosine kinase receptor and an antibody selected from nivolumab, atezolizumab and avelumab.

4. The method according to anyone of claims 1 to 3, wherein the CXCL5 and CXCL7 protein expression level(s) is/are determined before any administration to the subject of an inhibitor of a tyrosine kinase receptor or a combination of a tyrosine kinase receptor and an antibody selected from nivolumab, atezolizumab and avelumab for treating the subject's renal cancer.

5. The method according to anyone of claims 1 to 4, wherein the biological sample is a plasma sample .

6. The method according to anyone of claims 1 to 5, wherein the inhibitor of a tyrosine kinase receptor is selected from sunitinib, axitinib, pazopanib and sorafenib.

7. The method according to claim 6, wherein, when the inhibitor of a tyrosine kinase receptor is sunitinib, the CXCL5 reference protein expression level is of about 100 pg/ml and the CXCL7 reference protein expression level is of about 250 ng/ml.

8. The method according to anyone of claims 1 to 7, wherein CXCL5 and CXCL7 protein expression level(s) identical to or below respective reference protein expression level(s) is/are indicative of a resistance of the subject to the inhibitor of a tyrosine kinase receptor or a combination of a tyrosine kinase receptor and an antibody selected from nivolumab, atezolizumab and avelumab, and wherein CXCL5 and CXCL7 protein expression level(s) above said reference protein expression level(s) is/are indicative of a sensitivity of the subject to the inhibitor of a tyrosine kinase receptor or a combination of the tyrosine kinase receptor and the antibody selected from nivolumab, atezolizumab and avelumab.

9. The method according to claim 8, which method comprises a step of determining, in a biological sample from said subject, the respective protein expression levels of CXCL5 and CXCL7, and, when the CXCL5 and CXCL7 protein expression levels are determined, a step of comparing said CXCL5 and CXCL7 protein expression levels respectively to CXCL5 and CXCL7 reference protein expression levels, thereby assessing whether the subject is sensitive or resistant to the inhibitor of a tyrosine kinase receptor or a combination of the tyrosine kinase receptor and the antibody selected from nivolumab, atezolizumab and avelumab.

10. The method according to claim 9, wherein when the inhibitor of a tyrosine kinase receptor is sunitinib, a CXCL5 protein expression level identical to or below 100 pg/ml together with a CXCL7 protein expression level identical to or below 250 ng/ml is indicative of a resistance of the subject to sunitinib, and a CXCL5 protein expression level above 100 pg/ml together with a CXCL7 protein level above 250 ng/ml is indicative of a sensitivity of the subject to sunitinib.

11. A method of selecting, for a subject having a renal cancer, a treatment comprising an inhibitor of a tyrosine kinase receptor or a combination of a tyrosine kinase receptor and an antibody selected from nivolumab, atezolizumab and avelumab efficient against renal cancer in the subject, wherein the method comprises a step a) of determining the CXCL5 protein expression level or the CxCL5 and CXCL7 protein expression level(s) in a biological sample of the subject having renal cancer before any administration to the subject of an inhibitor of a tyrosine kinase receptor or a combination of a tyrosine kinase receptor and an antibody selected from nivolumab, atezolizumab and avelumab, wherein the biological sample is selected from a blood sample, a serum sample, a plasma sample and a step b) of comparing said CXCL5 and CXCL7 protein expression level(s) to (CXCL5 and CXCL7) reference protein expression level(s), CXCL5 and CXCL7 protein expression level(s) identical to the (CXCL5 and CXCL7) reference protein expression level(s) or below the (CXCL5 and CXCL7) reference protein expression level(s), being the indication that an inhibitor of a tyrosine kinase receptor or a combination of a tyrosine kinase receptor and an antibody selected from nivolumab, atezolizumab and avelumab will not be efficient, or will not be efficient alone in the subject, and a step c) of selecting an appropriate treatment of renal cancer in the subject, for example combining said inhibitor of a tyrosine kinase receptor or combination of the said tyrosine kinase receptor and the said antibody selected from nivolumab, atezolizumab and avelumab with an additional compound such as a CXCL5 or CXCL7 antibody; and, on the contrary, a CXCL5 and CXCL7 protein expression level(s) above the (CXCL5 and CXCL7) reference protein expression level(s) being the indication that the inhibitor of a tyrosine kinase receptor or the combination of the inhibitor of a tyrosine kinase receptor and the antibody selected from nivolumab, atezolizumab and avelumab will be efficient alone against renal cancer in the subj ect.

12. The method according to anyone of claim 1 to 11, wherein the subject is a subject who has undergone at least partial resection of the cancerous tumor.

13. The method according to anyone of claims 1 to 12, wherein the renal cancer is a metastatic clear cell renal cancer (ccRCC).

14. Use of a kit in an *in vitro* method according to anyone of claims 1 to 13 i) for assessing or monitoring the sensitivity or resistance of a subject having a renal cancer to an inhibitor of a tyrosine kinase receptor selected from sunitinib, axitinib, pazopanib, and sorafenib or a combination of a tyrosine kinase receptor selected from sunitinib, axitinib, pazopanib, and sorafenib and an antibody selected from nivolumab, atezolizumab and avelumab, and/or ii) for determining the potential toxicity of said inhibitor of a tyrosine kinase receptor or combination of the said tyrosine kinase receptor and the said antibody selected from nivolumab, atezolizumab and avelumab in a subject having renal cancer, wherein the kit comprises detection means consisting of at least CXCL5 polypeptide binding agent and CXCL7 polypeptide binding agent; a molecule allowing the binding agent detection; and, optionally, a leaflet providing the CXCL5 and the CXCL7 respective reference protein expression level(s).

## Patentansprüche

1. *In-vitro*-Verfahren zur Beurteilung der Empfindlichkeit eines Patienten mit Nierenkrebs gegenüber einem Hemmer eines Tyrosinkinase-Rezeptors zur Behandlung von Nierenkrebs, wobei das Verfahren einen Schritt a) des Bestimmens des Proteinexpressionsniveaus von CXCL5 in einer biologischen Probe dieses Patienten umfasst, und wenn das CXCL5-Proteinexpressionsniveau bestimmt ist, einen Schritt b) des Vergleichens des CXCL5-Proteinexpressionsniveaus mit einem CXCL5-Referenz-Proteinexpressionsniveau umfasst, wodurch beurteilt wird, ob der Patient mit ccRCC empfindlich oder resistent gegen den Hemmer eines Tyrosinkinase-Rezeptors ist, wobei die biologische Probe ausgewählt wird aus einer Blutprobe, einer Serumprobe, einer Plasmaprobe, wobei ein CXCL5-Proteinexpressionsniveau über dem Referenz-Proteinexpressionsniveau auf eine Empfindlichkeit des Patienten gegenüber dem Hemmer eines Tyrosinkinase-Rezeptors hindeutet.

2. Verfahren nach Anspruch 1, wobei das Verfahren weiter einen Schritt a') des Bestimmens des Proteinexpressionsniveaus von CXCL7 in einer biologischen Probe des Patienten umfasst und, wenn das CXCL7-Proteinexpressionsniveau bestimmt ist, einen Schritt b') des Vergleichens des CXCL7-Proteinexpressionsniveaus mit einem CXCL7-Referenz-Proteinexpressionsniveau umfasst.

3. Verfahren nach Anspruch 1 oder Anspruch 2 zur Beurteilung der Empfindlichkeit eines Patienten mit Nierenkrebs gegenüber einer chemotherapeutischen Kombination aus einem Hemmer eines Tyrosinkinase-Rezeptors und einem Antikörper ausgewählt aus Nivolumab, Atezolizumab und Avelumab.

4. Verfahren nach einem der Ansprüche 1 bis 3, wobei die CXCL5- und CXCL7-Proteinexpressionsniveaus vor jeder Verabreichung an den Patienten eines Hemmers eines Tyrosinkinase-Rezeptors oder einer Kombination eines Tyrosinkinase-Rezeptors und eines Antikörpers, ausgewählt aus Nivolumab, Atezolizumab und Avelumab, zur Behandlung des Nierenkrebses des Patienten bestimmt werden.

5. Verfahren nach einem der Ansprüche 1 bis 4, wobei die biologische Probe eine Plasmaprobe ist.

6. Verfahren nach einem der Ansprüche 1 bis 5, wobei der Hemmer eines Tyrosinkinase-Rezeptors ausgewählt wird aus Sunitinib, Axitinib, Pazopanib und Sorafenib.

7. Verfahren nach Anspruch 6, wobei der Hemmer eines Tyrosinkinase-Rezeptors Sunitinib ist, das CXCL5-Referenz-Proteinexpressionsniveau etwa 100 pg/ml und das CXCL7-Referenz-Proteinexpressionsniveau etwa 250 ng/ml beträgt.

8. Verfahren nach einem der Ansprüche 1 bis 7, wobei CXCL5- und CXCL7-Proteinexpressionsniveaus, die identisch mit den jeweiligen Referenz-Proteinexpressionsniveaus sind oder unter diesen liegen, auf eine Resistenz des Patienten gegenüber dem Hemmer eines Tyrosinkinase-Rezeptors oder einer Kombination aus einem Tyrosinkinase-Rezeptor und einem Antikörper, ausgewählt aus Nivolumab, Atezolizumab und Avelumab, hindeuten, und wobei CXCL5- und CXCL7-Proteinexpressionsniveaus über den Referenz-Proteinexpressionsniveaus auf eine Empfindlichkeit des Patienten gegenüber dem Hemmer eines Tyrosinkinase-Rezeptors oder einer Kombination aus dem Tyrosinkinase-Rezeptor und dem Antikörper, ausgewählt aus Nivolumab, Atezolizumab und Avelumab, hindeuten.

9. Verfahren nach Anspruch 8, wobei das Verfahren einen Schritt des Bestimmens der jeweiligen Proteinexpressionsniveaus von CXCL5 und CXCL7 in einer biologischen Probe des Patienten umfasst und, wenn die CXCL5- und CXCL7-Proteinexpressionswerte bestimmt sind, ein Schritt des Vergleichens der CXCL5- und CXCL7-Proteinexpressionsniveaus jeweils mit den CXCL5- und CXCL7-Referenz-Proteinexpressionsniveaus umfasst, wodurch beurteilt wird, ob der Patient empfindlich oder resistent gegen den Hemmer eines Tyrosinkinase-Rezeptors oder eine Kombination aus dem Tyrosinkinase-Rezeptor und dem Antikörper, ausgewählt aus Nivolumab, Atezolizumab und Avelumab, ist.

10. Verfahren nach Anspruch 9, wobei, wenn der Hemmer eines Tyrosinkinase-Rezeptors Sunitinib ist, ein CXCL5-Proteinexpressionsniveau, das gleich 100 pg/ml oder weniger ist, zusammen mit einem CXCL7-Proteinexpressionsniveau, das gleich 250 ng/ml oder weniger ist, auf eine Resistenz des Patienten gegen Sunitinib hindeutet, und ein CXCL5-Proteinexpressionsniveau über 100 pg/ml zusammen mit einem CXCL7-Proteinniveau über 250 ng/ml auf eine Empfindlichkeit des Patienten gegenüber Sunitinib hindeutet.

11. Verfahren zum Auswählen, für einen Patienten mit Nierenkrebs, einer Behandlung, die einen Hemmer eines Tyrosinkinase-Rezeptors oder eine Kombination eines Tyrosinkinase-Rezeptors und eines Antikörpers, ausgewählt aus Nivolumab, Atezolizumab und Avelumab, enthält, welche bei dem Patienten wirksam gegen Nierenkrebs ist, wobei das Verfahren einen Schritt a) umfasst des Bestimmens des CXCL5-Proteinexpressionsniveaus oder der CXCL5- und CXCL7-Proteinexpressionsniveaus in einer biologischen Probe des Patienten mit Nierenkrebs vor jeder Verabreichung an den Patienten eines Hemmers eines Tyrosinkinase-Rezeptors oder einer Kombination eines Tyrosinkinase-Rezeptors und eines Antikörpers ausgewählt aus Nivolumab, Atezolizumab und Avelumab, wobei die biologische Probe ausgewählt wird aus einer Blutprobe, einer Serumprobe, einer Plasmaprobe, und einen Schritt b) umfasst des Vergleichens der CXCL5- und CXCL7-Proteinexpressionsniveaus mit (CXCL5 und CXCL7-)Referenz-Proteinexpressionsniveaus, wobei CXCL5- und CXCL7-Proteinexpressionsniveaus identisch mit den (CXCL5- und CXCL7-)Referenz-Proteinexpressionsniveaus oder niedriger als die (CXCL5- und CXCL7-)Referenz-Proteinexpressionsniveaus darauf hindeuten, dass ein Hemmer eines Tyrosinkinase-Rezeptors oder eine Kombination eines Tyrosinkinase-Rezeptors und eines Antikörpers, ausgewählt aus Nivolumab, Atezolizumab und Avelumab, nicht oder nicht allein bei dem Patienten wirksam sein werden, und einen Schritt c) umfasst des Auswählens einer geeigneten Behandlung von Nierenkrebs bei dem Patienten, beispielsweise das Kombinieren des Hemmers eines Tyrosinkinase-Rezeptors oder der Kombination des Tyrosinkinase-Rezeptors und des Antikörpers, ausgewählt aus Nivolumab, Atezolizumab und Avelumab, mit einer zusätzlichen Verbindung, wie einem CXCL5- oder CXCL7-Antikörper; und wobei im Gegensatz dazu CXCL5- und CXCL7-Proteinexpressionsniveaus über den (CXCL5- und CXCL7-)Referenz-Proteinexpressionsniveaus darauf hindeuten, dass der Hemmer eines Tyrosinkinase-Rezeptors oder die Kombination des Hemmers eines Tyrosinkinase-Rezeptors und eines Antikörpers, ausgewählt aus Nivolumab, Atezolizumab und Avelumab, allein gegen Nierenkrebs bei dem Patienten wirksam sein wird.

12. Verfahren nach einem der Ansprüche 1 bis 11, wobei es sich bei dem Patienten um einen Patienten handelt, der sich mindestens einer Teilresektion des Krebstumors unterzogen hat.

13. Verfahren nach einem der Ansprüche 1 bis 12, wobei der Nierenkrebs ein metastasierender klarzelliger Nierenkrebs (ccRCC) ist.

14. Verwendung eines Kits in einem *in-vitro-*Verfahren nach einem der Ansprüche 1 bis 13 i) zur Beurteilung oder Überwachung der Empfindlichkeit oder Resistenz eines Patienten mit Nierenkrebs gegen einen Hemmer eines Tyrosinkinase-Rezeptors, ausgewählt aus Sunitinib, Axitinib, Pazopanib und Sorafenib, oder eine Kombination eines Tyrosinkinase-Rezeptors, ausgewählt aus Sunitinib, Axitinib, Pazopanib und Sorafenib, und eines Antikörpers, ausgewählt aus Nivolumab, Atezolizumab und Avelumab, und/oder ii) zur Bestimmung der potenziellen Toxizität des Hemmers eines Tyrosinkinase-Rezeptors oder einer Kombination des Tyrosinkinase-Rezeptors und des Antikörpers, ausgewählt aus Nivolumab, Atezolizumab und Avelumab, bei einem Patienten mit Nierenkrebs, wobei das Kit umfasst:
Nachweismittel, die aus mindestens CXCL5-Polypeptid-Bindemittel und CXCL7-Polypeptid-Bindemittel bestehen;
ein Molekül, das den Nachweis des Bindemittels ermöglicht; und optional
ein Beiblatt, das die CXCL5- und die CXCL7-Referenz-Proteinexpressionsniveaus bereitstellt.

## Revendications

1. Procédé *in vitro* de détermination de la sensibilité d'un sujet ayant un cancer rénal à un inhibiteur d'un récepteur de tyrosine kinase, pour le traitement d'un cancer rénal, lequel procédé comprend une étape a) de détermination, dans un échantillon biologique provenant dudit sujet, du niveau d'expression de la protéine CXCL5 et, quand le niveau d'expression de la protéine CXCL5 est déterminé, une étape b) de comparaison dudit niveau d'expression de la protéine CXCL5 avec un niveau d'expression de la protéine CXCL5 de référence, ce qui détermine ainsi si le sujet ayant un ccRCC est sensible ou résistant à l'inhibiteur d'un récepteur de tyrosine kinase, dans lequel l'échantillon biologique est choisi parmi un échantillon de sang, un échantillon de sérum, un échantillon de plasma, dans lequel un niveau d'expression de la protéine CXCL5 supérieur audit niveau d'expression de protéine de référence est indicatif d'une sensibilité du sujet à l'inhibiteur d'un récepteur de tyrosine kinase.

2. Procédé selon la revendication 1, lequel procédé comprend en outre une étape a') de détermination, dans un échantillon biologique provenant dudit sujet, du niveau d'expression de la protéine CXCL7 et, quand le niveau d'expression de la protéine CXCL7 est déterminé, une étape b') de comparaison dudit niveau d'expression de la protéine CXCL7 avec un niveau d'expression de la protéine CXCL7 de référence.

3. Procédé selon la revendication 1 ou la revendication 2, de détermination de la sensibilité d'un sujet ayant un cancer rénal à une combinaison chimiothérapeutique d'un inhibiteur d'un récepteur de tyrosine kinase et d'un anticorps choisi parmi le nivolumab, l'atézolizumab et l'avélumab.

4. Procédé selon l'une quelconque des revendications 1 à 3, dans lequel le ou les niveaux d'expression des protéines CXCL5 et CXCL7 sont déterminés avant toute administration au sujet d'un inhibiteur d'un récepteur de tyrosine kinase ou d'une combinaison d'un récepteur de tyrosine kinase et d'un anticorps choisi parmi le nivolumab, l'atézolizumab et l'avélumab pour le traitement du cancer rénal du sujet.

5. Procédé selon l'une quelconque des revendications 1 à 4, dans lequel l'échantillon biologique est un échantillon de plasma.

6. Procédé selon l'une quelconque des revendications 1 à 5, dans lequel l'inhibiteur d'un récepteur de tyrosine kinase est choisi parmi le sunitinib, l'axitinib, le pazopanib et le sorafénib.

7. Procédé selon la revendication 6, dans lequel, quand l'inhibiteur d'un récepteur de tyrosine kinase est le sunitinib, le niveau d'expression de la protéine CXCL5 de référence est d'environ 100 pg/ml et le niveau d'expression de la protéine CXCL7 de référence est d'environ 250 ng/ml.

8. Procédé selon l'une quelconque des revendications 1 à 7, dans lequel un ou des niveaux d'expression des protéines CXCL5 et CXCL7 identiques ou inférieurs aux niveaux d'expression de protéine de référence respectifs sont indicatifs d'une résistance du sujet à l'inhibiteur d'un récepteur de tyrosine kinase ou d'une combinaison d'un récepteur de tyrosine kinase et d'un anticorps choisi parmi le nivolumab, l'atézolizumab et l'avélumab, et dans lequel un ou des niveaux d'expression des protéines CXCL5 et CXCL7 supérieurs aux niveaux d'expression de protéine de référence respectifs sont indicatifs d'une sensibilité du sujet à l'inhibiteur d'un récepteur de tyrosine kinase ou d'une combinaison d'un récepteur de tyrosine kinase et d'un anticorps choisi parmi le nivolumab, l'atézolizumab et l'avélumab.

9. Procédé selon la revendication 8, lequel procédé comprend une étape de détermination, dans un échantillon biologique provenant dudit sujet, des niveaux d'expression des protéines CXCL5 et CXCL7 respectifs et, quand les niveaux d'expression des protéines CXCL5 et CXCL7 sont déterminés, une étape de comparaison desdits niveaux d'expression des protéines CXCL5 et CXCL7 respectivement avec des niveaux d'expression des protéines CXCL5 et CXCL7 de référence, ce qui détermine ainsi si le sujet sensible ou résistant à l'inhibiteur d'un récepteur de tyrosine kinase ou à une combinaison du récepteur de tyrosine kinase et de l'anticorps choisi parmi le nivolumab, l'atézolizumab et l'avélumab.

10. Procédé selon la revendication 9, dans lequel, quand l'inhibiteur d'un récepteur de tyrosine kinase est le sunitinib, un niveau d'expression de la protéine CXCL5 identique ou inférieur à 100 pg/ml, conjointement avec un niveau d'expression de la protéine CXCL7 identique ou inférieur à 250 ng/ml, sont indicatifs d'une résistance du sujet au sunitinib, et un niveau d'expression de la protéine CXCL5 supérieur à 100 pg/ml conjointement à un niveau d'expression de la protéine CXCL7 supérieur à 250 ng/ml sont indicatifs d'une sensibilité du sujet au sunitinib.

11. Procédé pour sélectionner, pour un sujet ayant un cancer rénal, un traitement comprenant un inhibiteur d'un récepteur de tyrosine kinase ou une combinaison d'un récepteur de tyrosine kinase et d'un anticorps choisi parmi le nivolumab, l'atézolizumab et l'avélumab, efficace contre un cancer rénal chez le sujet, lequel procédé comprend une étape a) de détermination du niveau d'expression de la protéine CXCL5 ou du ou des niveaux d'expression des protéines CXCL5 et CXCL7 dans un échantillon biologique du sujet ayant un cancer rénal avant toute administration au sujet d'un inhibiteur d'un récepteur de tyrosine kinase ou d'une combinaison d'un récepteur de tyrosine kinase et d'un anticorps choisi parmi le nivolumab, l'atézolizumab et l'avélumab, dans lequel l'échantillon biologique est choisi parmi un échantillon de sang, un échantillon de sérum, un échantillon de plasma, et une étape b) de comparaison dudit ou desdits niveaux d'expression des protéines CXCL5 et CXCL7 au ou aux niveaux d'expression de protéines de référence (CXCL5 et CXCL7), un ou des niveaux d'expression des protéines CXCL5 et CXCL7 identiques au ou aux niveaux d'expression de protéines de référence (CXCL5 et CXCL7) ou inférieurs au ou aux niveaux d'expression de protéines de référence (CXCL5 et CXCL7) étant une indication qu'un inhibiteur de tyrosine kinase ou une combinaison d'un récepteur de tyrosine kinase et d'un anticorps choisi parmi le nivolumab, l'atézolizumab et l'avélumab ne va pas être efficace, ou ne va pas être efficace seul chez le sujet, et une étape c) de sélection d'un traitement approprié du cancer rénal chez le sujet, par exemple une combinaison dudit inhibiteur d'un récepteur de tyrosine kinase ou une combinaison dudit récepteur de tyrosine kinase et dudit anticorps choisi parmi le nivolumab, l'atézolizumab et l'avélumab avec un composé additionnel tel qu'un anticorps anti-CXCL5 ou anti-CXCL7 ; et, au contraire, un ou des niveaux d'expression des protéines CXCL5 et CXCL7 supérieurs au ou aux niveaux d'expression de protéines de référence (CXCL5 et CXCL7) étant une indication que l'inhibiteur d'un récepteur de tyrosine kinase ou la combinaison de l'inhibiteur d'un récepteur de tyrosine kinase et de l'anticorps choisi parmi le nivolumab, l'atézolizumab et l'avélumab va être efficace seul contre le cancer rénal chez le sujet.

12. Procédé selon l'une quelconque des revendications 1 à 11, dans lequel le sujet est un sujet qui a subi au moins une résection partielle de la tumeur cancéreuse.

13. Procédé selon l'une quelconque des revendications 1 à 12, dans lequel le cancer rénal est un cancer rénal à cellules claires métastasiques (ccRCC).

14. Utilisation d'un kit dans un procédé *in vitro* selon l'une quelconque des revendications 1 à 13 i) pour déterminer ou surveiller la sensibilité ou la résistance d'un sujet ayant un cancer rénal à un inhibiteur d'un récepteur de tyrosine kinase choisi parmi le sunitinib, l'axitinib, le pazopanib et le sorafénib ou à une combinaison d'un inhibiteur d'un récepteur de tyrosine kinase choisi parmi le sunitinib, l'axitinib, le pazopanib et le sorafénib et d'un anticorps choisi parmi le nivolumab, l'atézolizumab et l'avélumab, et/ou ii) pour déterminer la toxicité potentielle dudit inhibiteur d'un récepteur de tyrosine kinase ou de la combinaison dudit inhibiteur d'un récepteur de tyrosine kinase et dudit anticorps choisi parmi le nivolumab, l'atézolizumab et l'avélumab chez un sujet ayant un cancer rénal, dans laquelle le kit comprend des moyens de détection consistant en au moins un agent de liaison à CXCL5 polypeptidique et un agent de liaison à CXCL7 polypeptidique ; une molécule permettant la détection de l'agent de liaison ; et éventuellement une notice indiquant le ou les niveaux d'expression des protéines CXCL5 et CXCL7 de référence respectifs.


### SEQUENCE LISTING

<110> CNRS et al.
<120> Methods and kits for predicting the sensitivity of a subject suffering of renal cancer to cancer treatment
<130> B2222PC00
<160> 14
<170> PatentIn version 3.5
<210> 1
   <211> 114
   <212> PRT
   <213> Homo sapiens
<400> 1
   Met Ser Leu Leu Ser Ser Arg Ala Ala Arg Val Pro Gly Pro Ser Ser
   1               5                   10                  15
   Ser Leu Cys Ala Leu Leu Val Leu Leu Leu Leu Leu Thr Gln Pro Gly
               20                  25                  30
   Pro Ile Ala Ser Ala Gly Pro Ala Ala Ala Val Leu Arg Glu Leu Arg
           35                  40                  45
   Cys Val Cys Leu Gln Thr Thr Gln Gly Val His Pro Lys Met Ile Ser
       50                  55                  60
   Asn Leu Gln Val Phe Ala Ile Gly Pro Gln Cys Ser Lys Val Glu Val
   65                  70                  75                  80
   Val Ala Ser Leu Lys Asn Gly Lys Glu Ile Cys Leu Asp Pro Glu Ala
                   85                  90                  95
   Pro Phe Leu Lys Lys Val Ile Gln Lys Ile Leu Asp Gly Gly Asn Lys
               100                 105                 110
   Glu Asn
<210> 2
   <211> 1307
   <212> DNA
   <213> Homo sapiens
<400> 2
   acttatctgc agacttgtag gcagcaactc accctcactc agaggtcttc tggttctgga        60
   aacaactcta gctcagcctt ctccaccatg agcctcagac ttgataccac cccttcctgt     120
   aacagtgcga gaccacttca tgccttgcag gtgctgctgc ttctgtcatt gctgctgact     180
   gctctggctt cctccaccaa aggacaaact aagagaaact tggcgaaagg caaagaggaa      240
   agtctagaca gtgacttgta tgctgaactc cgctgcatgt gtataaagac aacctctgga      300
   attcatccca aaaacatcca aagtttggaa gtgatcggga aaggaaccca ttgcaaccaa      360
   gtcgaagtga tagccacact gaaggatggg aggaaaatct gcctggaccc agatgctccc      420
   agaatcaaga aaattgtaca gaaaaaattg gcaggtgatg aatctgctga ttaatttgtt      480
   ctgtttctgc caaacttctt taactcccag gaagggtaga attttgaaac cttgattttc      540
   tagagttctc atttattcag gatacctatt cttactgtat taaaatttgg atatgtgttt      600
   cattctgtct caaaaatcac attttattct gagaaggttg gttaaaagat ggcagaaaga      660
   agatgaaaat aaataagcct ggtttcaacc ctctaattct tgcctaaaca ttggactgta      720
   ctttgcattt ttttctttaa aaatttctat tctaacacaa cttggttgat ttttcctggt      780
   ctactttatg gttattagac atactcatgg gtattattag atttcataat ggtcaatgat      840
   aataggaatt acatggagcc caacagagaa tatttgctca atacattttt gttaatatat      900
   ttaggaactt aatggagtct ctcagtgtct tagtcctagg atgtcttatt taaaatactc      960
   cctgaaagtt tattctgatg tttattttag ccatcaaaca ctaaaataat aaattggtga     1020
   atatgaatct tataaactgt ggttagctgg tttaaagtga atatatttgc cactagtaga     1080
   acaaaaatag atgatgaaaa tgaattaaca tatctacata gttataattc tatcattaga     1140
   atgagcctta taaataagta caatatagga cttcaacctt actagactcc taattctaaa     1200
   ttctactttt ttcatcaaca gaactttcat tcatttttta aaccctaaaa cttataccca      1260
   cactattctt acaaaaatat tcacatgaaa taaaaatttg ctattga                  1307
<210> 3
   <211> 11
   <212> PRT
   <213> Artificial Sequence
<220>
   <223> Light chain CDR1 (LC-CDR1) - hybridoma 35B11-8
<400> 3
   Gln Ala Ser Gln Asn Ile Asn Lys Tyr Ile Ala
   1               5                   10
<210> 4
   <211> 6
   <212> PRT
   <213> Artificial Sequence
<220>
   <223> Light chain CDR2 (LC-CDR2) - hybridoma 35B11-8
<400> 4
   Thr Ser Thr Leu Val Ser
   1 5
<210> 5
   <211> 9
   <212> PRT
   <213> Artificial Sequence
<220>
   <223> Light chain CDR3 (LC-CDR3) - hybridoma 35B11-8
<400> 5
   Leu Gln Tyr Asp Asn Ser Pro Phe Pro
   1 5
<210> 6
   <211> 9
   <212> PRT
   <213> Artificial Sequence
<220>
   <223> Heavy chain CDR1 (HC-CDR1) - hybridoma 35B11-8
<400> 6
   Gly Phe Thr Phe Asn Asn Tyr Trp Met
   1 5
<210> 7
   <211> 17
   <212> PRT
   <213> Artificial Sequence
<220>
   <223> Heavy chain CDR2 (HC-CDR2) - hybridoma 35B11-8
<400> 7
   Ser Ile Ser Lys Thr Gly Ser Asn Pro Tyr Tyr Pro Asp Ser Val Lys
   1 5 10 15
   Gly
<210> 8
   <211> 14
   <212> PRT
   <213> Artificial Sequence
<220>
   <223> Heavy chain CDR3 (HC-CDR3) - hybridoma 35B11-8
<400> 8
   Asp Gly Thr Thr Pro Leu Asp Tyr Trp Gly Gln Gly Val Met
   1 5 10
<210> 9
   <211> 10
   <212> PRT
   <213> Artificial Sequence
<220>
   <223> Light chain CDR1 (LC-CDR1) - hybridoma 12A10-13
<400> 9
   Arg Ala Ser Ser Ser Val Ser Tyr Met His
   1               5                   10
<210> 10
   <211> 6
   <212> PRT
   <213> Artificial Sequence
<220>
   <223> Light chain CDR2 (LC-CDR2) - hybridoma 12A10-13
<400> 10
   Thr Ser Lys Leu Ala Ser
   1               5
<210> 11
   <211> 9
   <212> PRT
   <213> Artificial Sequence
<220>
   <223> Light chain CDR3 (LC-CDR3) - hybridoma 12A10-13
<400> 11
   Gln Gln Trp Ser Ser Asn Ser Leu Thr
   1               5
<210> 12
   <211> 9
   <212> PRT
   <213> Artificial Sequence
<220>
   <223> Heavy chain CDR1 (HC-CDR1) - hybridoma 12A10-13
<400> 12
   Gly Tyr Thr Phe Thr Ser Tyr Tyr Ile
   1               5
<210> 13
   <211> 17
   <212> PRT
   <213> Artificial Sequence
<220>
   <223> Heavy chain CDR2 (HC-CDR2) - hybridoma 12A10-13
<400> 13
   Tyr Ile Asn Thr Gly Ser Gly Gly Thr Asn Tyr Asn Glu Lys Phe Lys
   1               5                   10                  15
   Gly
<210> 14
   <211> 13
   <212> PRT
   <213> Artificial Sequence
<220>
   <223> Heavy chain CDR3 (HC-CDR3) - hybridoma 12A10-13
<400> 14
   Pro Ser Gly Gly Phe Asp Tyr Trp Gly Gln Gly Val Met
   1               5                   10